(19)

Europäisches Patentamt

European Patent Office

Office européen des brevets

(11)    **EP 4 545 002 A1**

(12)    **EUROPEAN PATENT APPLICATION**

(43) Date of publication:
30.04.2025   Bulletin 2025/18

(21) Application number: 24208161.0

(22) Date of filing: **22.10.2024**

(51) International Patent Classification (IPC):
**A61B 5/145** *(2006.01)*      **A61B 5/00** *(2006.01)*
**A61B 5/1455** *(2006.01)*      **A61B 5/1495** *(2006.01)*

(52) Cooperative Patent Classification (CPC):
**A61B 5/14532; A61B 5/7235;** A61B 5/1451;
A61B 5/1455; A61B 5/14865; A61B 5/1495;
A61B 5/4839

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **27.10.2023   PCT/GR2023/000057
08.10.2024   US 202418908980**

(71) Applicant: **Medtronic MiniMed, Inc.
Northridge, CA 91325 (US)**

(72) Inventors:
• **Basilico, Jake G.
Northridge, 91325 (US)**
• **Nava-Gurerra, Leonardo
Northridge, 91325 (US)**

• **Notghi, Bahram B.
Northridge, 91325 (US)**
• **Emig, Molly E.
Northridge, 91325 (US)**
• **Liu, Jing
Northridge, 91325 (US)**
• **Willats, Adam A.
Northridge, 91325 (US)**
• **Mallas, Georgios
Northridge, 91325 (US)**
• **Zhang, Yi
Northridge, 91325 (US)**

(74) Representative: **Zimmermann & Partner
Patentanwälte mbB
Postfach 330 920
80069 München (DE)**

(54)    **ENSEMBLE OF PARTITIONED SENSOR GLUCOSE MODELS**

(57)    A processor-implemented method includes receiving sensor measurement data from a glucose sensor; selecting, based on the sensor measurement data, a first regional sensor glucose (SG) model from a first plurality of regional SG models for respective regions of a first plurality of regions of an input parameter space associated with the sensor measurement data, and a second regional SG model from a second plurality of regional SG models for respective regions of a second plurality of regions of the input parameter space; estimating a first SG value and a second SG value using the first regional SG model and the second regional SG model, respectively; and determining a predicted SG value based on a combination of the first SG value and the second SG value. The input parameter space is partitioned into the first plurality of regions and the second plurality of regions using different partition schemes.

*FIG. 8*

**EP 4 545 002 A1**

**Description**

TECHNICAL FIELD

The present disclosure relates generally to glucose level measurement and/or management.

BACKGROUND

The pancreas of a healthy person can produce and release insulin into the blood stream in response to elevated blood glucose levels. More specifically, beta cells ($\beta$-cells) in the pancreas can produce and secrete insulin into the blood stream as needed. If $\beta$-cells become incapacitated or die, a condition known as Type 1 diabetes mellitus, insulin may need to be provided to the diabetic patient's body using, for example, a syringe, a pen, or an infusion pump of a blood glucose level management system, to maintain health or life.

Some blood glucose level management systems may be closed-loop systems that may include a pump automatically or semi-automatically controlled to deliver insulin to the patient. Some blood glucose level management systems may require manual administration of insulin based on dosage determined by an insulin calculator. The delivery of insulin to the patient can be controlled to occur at time instants and in amounts that are determined based on, for example, the amount of carbohydrates taken by a patient and/or real-time measurements of glucose levels by a glucose sensor. Glucose sensors may detect and/or quantify the amount of glucose in a patient's body (e.g., interstitial glucose or blood glucose), which enables patients and medical personnel to monitor physiological conditions within the patient's body. In many circumstances (e.g., in a diabetic patient), it may be beneficial to monitor glucose levels on a continuing basis. Such information can be useful in monitoring and/or adjusting a treatment regimen, which typically includes administration of insulin to the patient. Rather than continuously sampling and monitoring a user's blood glucose level, which may compromise battery life, intermittently sensed glucose data samples may often be utilized for purposes of continuous glucose monitoring (CGM) or determining operating commands for the infusion pump.

SUMMARY

This disclosure relates generally to glucose level measurement and/or management. More specifically, techniques disclosed herein relate to estimating blood glucose levels using measurement data from a subcutaneous sensor and an ensemble of partitioned sensor glucose models. The techniques disclosed herein may be practiced in a variety of ways, such as using a processor-implemented method, a system comprising one or more processors and one or more processor-readable media, and/or one or more (non-transitory) processor-readable media.

According to certain embodiments, the techniques may involve: receiving sensor measurement data measured by a glucose sensor, the sensor measurement data including at least one signal that is indicative of glucose level and at least one signal that is indicative of sensor health; selecting, based at least in part on the sensor measurement data, a first regional sensor glucose (SG) model from a first plurality of regional SG models for respective regions of a first plurality of regions of an input parameter space associated with the sensor measurement data, wherein the input parameter space is partitioned into the first plurality of regions based on a first partition scheme; estimating a first SG value using the first regional SG model and the sensor measurement data; selecting, based at least in part on the sensor measurement data, a second regional SG model from a second plurality of regional SG models for respective regions of a second plurality of regions of the input parameter space, wherein the input parameter space is partitioned into the second plurality of regions based on a second partition scheme that is different from the first partition scheme; estimating a second SG value using the second regional SG model and the sensor measurement data; and determining a predicted SG value based on a combination of the first SG value and the second SG value.

This summary is neither intended to identify key or essential features of the claimed subject matter, nor is it intended to be used in isolation to determine the scope of the claimed subject matter. The subject matter should be understood by reference to appropriate portions of the entire specification of this disclosure, any or all drawings, and each claim. The foregoing, together with other features and examples, will be described in more detail below in the following specification, claims, and accompanying drawings.

Further disclosed herein is a processor-implemented method which includes receiving sensor measurement data from a glucose sensor; selecting, based on the sensor measurement data, a first regional sensor glucose (SG) model from a first plurality of regional SG models for respective regions of a first plurality of regions of an input parameter space associated with the sensor measurement data, and a second regional SG model from a second plurality of regional SG models for respective regions of a second plurality of regions of the input parameter space; estimating a first SG value and a second SG value using the first regional SG model and the second regional SG model, respectively; and determining a predicted SG value based on a combination of the first SG value and the second SG value. The input parameter space is partitioned into the first plurality of regions and the second plurality of regions using different partition schemes.

BRIEF DESCRIPTION OF THE DRAWINGS

Illustrative embodiments are described in detail below with reference to the following figures.

FIG. 1 illustrates an example of a blood glucose level management system according to certain embodiments.

FIG. 2 is a block diagram of an example of a glucose control system according to certain embodiments.

FIG. 3 illustrates an example of a continuous glucose monitoring (CGM) system according to certain embodiments.

FIG. 4A illustrates an example of a glucose sensor according to certain embodiments.

FIG. 4B illustrates another example of a glucose sensor according to certain embodiments.

FIG. 4C illustrates a cross-section of an example of a working electrode of a glucose sensor according to certain embodiments.

FIG. 5 shows a schematic of a glucose sensor according to certain embodiments.

FIG. 6 includes a flow diagram illustrating an example of a method of determining sensor glucose values using measurement data from a glucose sensor, in accordance with certain embodiments.

FIG. 7 illustrates an example of a sensor feature generator according to certain embodiments.

FIG. 8 illustrates an example of determining a sensor glucose value using one or more models according to certain embodiments.

FIG. 9A shows an example of a partitioned model including a set of regional models for modeling a relationship between sensor glucose (SG) values and sensor measurement data according to certain embodiments.

FIG. 9B shows another example of a partitioned model including a set of regional models for modeling a relationship between SG values and sensor measurement data according to certain embodiments.

FIG. 10A shows an example of a partitioned model including a set of regional models for modeling a relationship between SG values and sensor measurement data according to certain embodiments.

FIG. 10B shows another example of a partitioned model including a set of regional models for modeling a relationship between SG values and sensor measurement data according to certain embodiments.

FIG. 11 illustrates an example of estimating SG values using an ensemble of multiple sets of regional models according to certain embodiments.

FIG. 12A illustrates an example of an ensemble model including two sets of regional SG models for estimating SG values based on sensor measurement data generated by a glucose sensor according to certain embodiments.

FIG. 12B illustrates examples of performance improvement over a baseline technique using techniques disclosed herein according to certain embodiments.

FIG. 13 includes a flowchart illustrating an example of a process of estimating SG values using an ensemble of partitioned SG models according to certain embodiments.

FIGS. 14A and 14B depict an example of an integrated glucose sensor device according to certain embodiments, which may implement some of the examples disclosed herein.

FIG. 15 depicts an example of a delivery device, which may implement some of the examples disclosed herein.

FIG. 16 is a simplified block diagram of an example of an electronic device that may implement some of the examples disclosed herein.

The figures depict embodiments of the present disclosure for purposes of illustration only. One skilled in the art will readily recognize from the following description that alternative embodiments of the structures and methods illustrated may be employed without departing from the principles, or benefits touted, of this disclosure.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

DETAILED DESCRIPTION

This disclosure relates generally to glucose level measurement and/or management. More specifically, techniques disclosed herein relate to estimating blood glucose levels using measurement data from a subcutaneous glucose sensor and an ensemble of partitioned sensor glucose models. The techniques disclosed herein may be practiced in a variety of ways, such as using a processor-implemented method, a system comprising one or more processors and one or more processor-readable media, and/or one or more (non-transitory) processor-readable media.

Diabetes mellitus is a disease of the glucose regulatory system of a patient, where the naturally produced insulin in the body may not be sufficient to control the glucose level in the blood stream of the patient, due to insufficient production of insulin and/or insulin resistance. Therefore, a diabetic patient may need to receive insulin from a pump or another delivery device, such as an injection or infusion device (e.g., a syringe), to control the glucose level in the patient's blood stream. To control the glucose level, a diabetic patient's therapy routine may generally include dosages of basal insulin and bolus insulin. Basal insulin, also referred to as background insulin, may include continuous or constant release of small amounts of insulin to keep blood glucose levels at consistent levels during long time periods. Bolus insulin may be taken specifically before, at, or after mealtimes or other times where there may be a rapid increase in the blood glucose level.

The dose of insulin to be delivered may be determined based on, for example, the carbohydrate count of a meal, and/or the glucose levels of the patient measured using a glucose monitor, such as a fingerstick blood glucose measurement device or a continuous glucose monitoring (CGM) sensor. A CGM sensor may use measurement results (e.g., currents, voltage levels, resistance, etc.) from a sensor inserted into the subcutaneous layer of a user to estimate the blood glucose level of the user based on the glucose level in the interstitial fluid (ISF). In some implementations, a glucose sensor may produce signals through a series of electrochemical reactions when the sensor is inserted and exposed to glucose and oxygen diffused from the interstitial fluid. For example, a glucose sensor may include a glucose limiting membrane (GLM) that limits the amount of glucose and oxygen delivered to a glucose oxidase (GOx) layer of a working electrode of the glucose sensor to ensure that the reactions are glucose limited. The GOx layer or another active enzyme layer on the working electrode of the glucose sensor may break down glucose and oxygen into gluconic acid and hydrogen peroxide. The generated hydrogen peroxide molecules may interact with the working electrode (e.g., with a voltage level applied) to break down hydrogen peroxide into two hydrogen ions, oxygen, and two electrons at the surface of the working electrode. The electrical charges may be forced to move between electrodes due to the potential difference, thereby generating a sensor current signal (Isig) that can be measured by sensor electronics. Other signals such as counter voltage (Vcntr, the voltage potential difference between the counter electrode and the working electrode), electrochemical impedance spectroscopy (EIS) at different frequencies, and the like, may also be measured. The signals measured using the sensor, including Isig, Vcntr, and EIS, may be processed (e.g., filtered, calibrated, or otherwise transformed) to generate some other signals or parameters, such as filtered Isig signals, real and imaginary impedances at various frequencies, derivative signals, transformed signals, and the like. The raw signals measured using the sensor and/or the processed signals may be used in one or more sensor glucose (SG) models to determine SG values that may be estimations of the blood glucose (BG) levels of the user.

The one or more SG models may include one or more trained machine learning models, equations, functions, and the like that may map input parameters (e.g., raw and/or processed sensor measurement data) to SG (or BG) values. For example, an SG model may be represented by:

$$SG = f(Age, Isig, Vcntr, EIS),$$

where age may be the time of the glucose sensor in operation after the insertion. In a simplified example, SG values may be calculated according to:

$$SG = \alpha \times Age + \beta \times Isig + \gamma \times Vcntr + \delta \times EIS + \Delta,$$

where $\alpha$, $\beta$, $\gamma$, and $\delta$ may be coefficients associated with the age, Isig, Vcntr, and EIS, and $\Delta$ is an offset value. In some embodiments, at least some of the coefficients may include vectors or tensors, and at least some of the age, Isig, Vcntr, and EIS may include vectors or tensors.

To achieve the desired accuracy and reliability and reduce the impact of noise and other spurious signals, CGM sensors may need to be periodically or occasionally calibrated to improve the accuracy and/or reliability of the glucose sensors. For example, the sensor data may need to be calibrated using a known good blood glucose value, often obtained via a so-called "fingerstick measurement" using a blood glucose meter that measures the blood glucose level in the capillaries. Performing such calibration measurements may increase the patient's burden and perceived complexity, and can be inconvenient, uncomfortable, or otherwise disfavored by patients. Moreover, ISF glucose measurements may lag behind the blood glucose measurements due to the time it takes glucose to diffuse from the capillary to the interstitial space, which may require certain signal processing (e.g., filtering) or other techniques to compensate for the physiological lag. Additionally, various factors can lead to transient changes in the sensor output, which may influence the accuracy of the calibration and the measurement. Degradation of sensor performance over time and manufacture variations between the sensors may further compound these challenges.

Sensor properties associated with a glucose sensor, such as the age (or time of use or wear time), battery level, calibration accuracy, sensing environment (e.g., oxygen level, temperature, moisture, and pH value, etc.), physiological dynamics, manufacturing variability, and other properties, may affect the enzymatic glucose level measurement results in complex ways that may be difficult to model. Therefore, many CGM sensors may have suboptimal accuracy and may exhibit large variance in the measurement results. The lower than desired performance of the CGM sensors may be at least partially attributed to the complexity of modeling the in vivo behavior of the CGM sensors, in addition to the sensor-to-sensor manufacturing variability, degradation of the sensor performance over time, and the like. In many cases, a single SG model may not be able to accurately estimate the blood glucose level, or the single SG model may be complicated and difficult to train, because various sensor properties may affect the glucose level measurement results in complex ways that may be difficult to characterize.

According to certain embodiments, different SG models may be used for different input parameter ranges (e.g., different subspaces of the input parameter space of the SG models), such as different ranges of the age, Vcntr, Isig, EIS, or other sensor measurement data. Each SG model may be optimized for a respective region of the input parameter space (referred to herein as regional SG model or regional model), and thus may be able to more accurately characterize the relationship between the glucose level and the sensor measurement data within the respective region. Multiple sets of regional SG models may be generated and used to determine SG values that approximate the blood glucose levels of a user, where the different sets of regional SG models may be generated for different partitioning of the input parameter space (e.g., different combinations of input parameters ranges). The input parameters may include, for example, Isig, Vcntr, EIS, age, sensor sensitivity, and the like. The input parameter space may be divided differently according to different partition schemes, for example, based on different combinations of input parameters, and/or by dividing the ranges of a same combination of input parameters differently. A set of regional SG models may be generated for regions of the input parameter space divided according to a respective partition scheme. One regional SG model may be selected from each set of regional SG models for a respective input parameter space partition scheme, based on the region in which the current input parameters fall. Multiple regional SG models selected from the multiple sets of regional SG models for the different partition schemes may be used to estimate the SG values independently, and then the outputs of the selected multiple regional SG models may be combined (e.g., by averaging or weighted averaging) to determine a predicted SG value.

Using the ensemble of multiple sets of regional SG models disclosed herein may more accurately model the sensor variance (e.g., sensitivity variations), and may significantly improve the performance (e.g., accuracy and consistency) of CGM sensors. Each regional model may be trained independently at a faster speed using a smaller set of training data, and may better characterize the response of the glucose sensor to analyte of different concentrations, such as the sensitivity and the nonlinearity of the sensitivity. Using certain parameters such as Isig as additional parameters for partitioning the input parameter space may also help to improve the performance of the SG models due to, for example, the improved granularity of partitioning the input parameter space and the relevancy of the additional parameters for modeling the response of the glucose sensor. Compared to using a one complex SG model or one set of regional SG models having a large number of models for a large number of regions partitioned using a large number of input parameters, using an ensemble of multiple sets of regional SG models for different partition schemes (e.g., using different input parameters for different partition schemes) may further improve the performance (e.g., accuracy and consistency) of CGM sensors due to, for example, the use of additional parameters to improve the granularity of the partitioning, and the use of additional partition schemes to include additional parameters or freedom for tuning and improving the SG models, without significantly increasing the overall complexity of the SG models and the difficulty of training the SG models.

Aspects and embodiments of the present disclosure can be practiced with one or more types of insulin (e.g., fast-acting insulin, intermediate-acting insulin, and/or slow-acting insulin). Unless indicated otherwise by the context, terms such as "dose," "insulin," "basal," and "bolus" may not denote a particular type of insulin. For example, fast-acting insulin may be used for both basal dosages and bolus dosages. The term "basal" can refer to and include insulin that is delivered in an amount and at a frequency that is intended to correspond to a healthy body's release of insulin between meals and during sleep. The term "bolus" may refer to and include insulin that is delivered in an amount and at a timing that is intended to correspond to a healthy body's release of insulin for counteracting a high glucose level, such as that caused by the consumption of a meal. A meal may include any type or amount of food or beverage consumption, including breakfast, lunch, dinner, snacks, and beverages, among others.

In the following description, for the purposes of explanation, specific details are set forth in order to provide a thorough understanding of examples of the disclosure. However, it will be apparent that various examples may be practiced without these specific details. For example, devices, systems, structures, assemblies, methods, and other components may be shown as components in block diagram form in order not to obscure the examples in unnecessary detail. In other instances, well-known devices, processes, systems, structures, operations, and techniques may be shown without necessary detail or may not be shown, in order to avoid obscuring the examples. The figures and description are not intended to be restrictive. The terms and expressions that have been employed in this disclosure are used as terms of description and not of limitation, and there is no intention in the use of such terms and expressions of excluding any equivalents of the features shown and described or portions thereof. The word "example" is used herein to mean "serving as an example, instance, or illustration." Any embodiment or design described herein as "example" is not necessarily to be construed as preferred or advantageous over other embodiments or designs.

Carbohydrates in food intake may be broken down into glucose (sugar) in the stomach and/or intestine, and may be absorbed at the small intestine and/or large intestine into the blood stream. The blood stream may transport glucose to the capillaries of the body, where some glucose may diffuse into the interstitial fluid between cells (e.g., fat or muscle cells), which may use the glucose for energy. The endocrine system of the human body that directs and regulates the functions and activities of the body (working with the nervous system) may secrete chemicals that transmit messages to tissues and organs. For example, endocrine glands or organs may release hormones into the blood stream for transportation to target cells with receptors. In one example, insulin may be made by the β-cells of pancreas, and may, for example, increase the glucose uptake, enhance glucose utilization, stop hepatic glucose production, stimulate glycogen formation in liver and skeletal muscle, promote protein synthesis, and increase fat storage.

Insulin may function as a key that can unlock cells and help glucose to move into cells where the glucose may be used for energy. Without insulin, glucose may not be able to enter cells to be used for energy, and may build up in the interstitial fluid and blood stream. A healthy pancreas may continuously release a small amount of regular human insulin 24 hours a day, including between meals and during sleep. The small amount pf insulin may match the liver's release of glucose. A healthy pancreas may also secrete a larger amount of insulin after food intake to match the amount of food intake.

The liver of a person may absorb excessive glucose from digestion and convert excessive glucose to glycogen for storage so that glucose may be available when needed. For example, when the blood glucose level is low, the α-cells of the pancreas may secrete glucagon. Glucagon may cause the liver to release the stored form of glucose (glycogen) into the blood stream to help increase the glucose level. The balance between insulin secreted by the β-cells and the glucagon secreted by the α-cells may help to maintain the normal blood glucose level, such as in the range of about 80-120 mg/dL before meals.

The naturally produced insulin in the body of a diabetic patient may not be sufficient to control the glucose level in the blood stream of the patient, due to insufficient production of insulin and/or insulin resistance. Therefore, a diabetic patient may need to receive insulin from a pump or another delivery device such as an injection or infusion device to control the glucose level in the patient's blood stream. To control the glucose level, a diabetic patient's therapy routine may generally include dosages of basal insulin and bolus insulin. Dosages of insulin to be delivered may be determined based on, for example, the carbohydrate count of a meal, and/or the glucose level of the patient measured using a glucose monitor, such as a continuous glucose monitor (CGM).

In some implementations (e.g., in a closed-loop system), the insulin delivery device may communicate with or otherwise use a sensor device (including but not limited to a CGM) to perform various measurements for a patient. In one example, the sensor device may include subcutaneous implanted electrodes to concurrently monitor the patient's response to meals and insulin introduced by the insulin delivery device. The sensor device and the insulin delivery device may be in a communication network (wired or wireless) with one or more processors and/or patient devices (such as a patient's smartphone equipped with an application or other software) to create an overall system for monitoring a patient disease state and for facilitating treatment thereof.

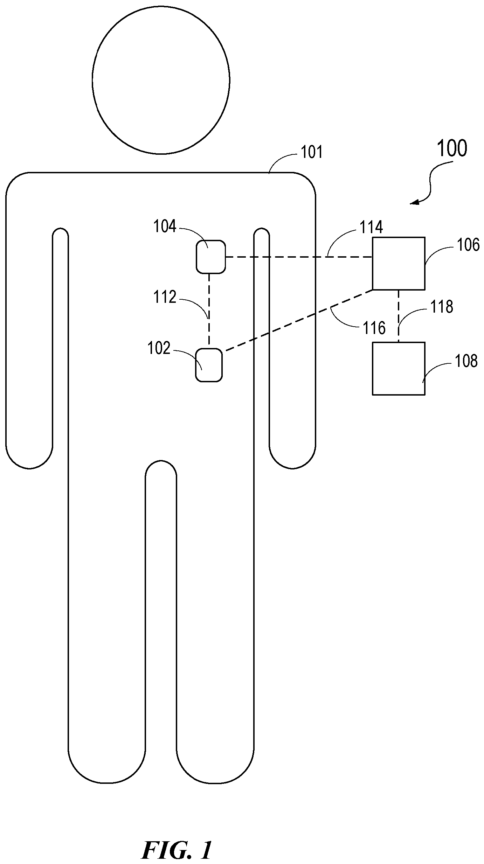

**FIG. 1** illustrates an example of a blood glucose level management system 100 according to certain embodiments. Blood glucose level management system 100 may be used to monitor and regulate the blood glucose level of a patient 101. In the illustrated example, blood glucose level management system 100 may include a delivery device 102, a monitoring device 104, a computing device 106, and an optional remote/cloud computing system 108. Delivery device 102, monitoring device 104, and computing device 106 may be embodied in various ways, including being disposed in one or more device housings. For example, in some embodiments, all of devices 102-106 may be disposed in a single device housing. In some embodiments, each of devices 102-106 may be disposed in a separate device housing. In some embodiments, two or more of devices 102-106 may be disposed in the same device housing. In some embodiments, a single device 102, 104, or 106 may have two or more parts that are disposed in two or more housings. For example, monitoring device 104 may include an on-body part and a display and control part communicated with the on-body part through wires or wirelessly. Delivery device 102 may include an on-body site (e.g., including a cannula) and a part that includes a reservoir, a pump, and a control unit. These and other embodiments, and combinations thereof, are contemplated to be within the scope of the present disclosure.

Blood glucose level management system 100 may include a plurality of communication links, such as communication links 112-118. Communications links 112-118 may each be a wired connection and/or a wireless connection. In embodiments where two devices are located in a same housing, the communication link may include, for example, wires, cables, and/or communication buses on a printed circuit board, among other things. In embodiments where two devices are separate from each other in different device housings, the communication links may be wired and/or wireless connections. Wired connections may include, for example, an Ethernet connection, a Universal Serial Bus (USB) connection, and/or another type of physical connection. Wireless connections may include, for example, a cellular connection, a Wi-Fi connection, a Bluetooth® connection, a mesh network connection, and/or another type of connection using a wireless communication protocol. Some embodiments of communication links 112-118 may use direct connections, such as Bluetooth® connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for communication links 112-118.

Delivery device 102 may be configured to deliver a therapeutic substance to patient 101. The therapeutic substance may include, for example, insulin, HIV drugs, drugs to treat pulmonary hypertension, iron chelation drugs, pain medications, anti-cancer treatments, medications, vitamins, hormones, a nutritional supplement, a dye, a tracing medium, a saline medium, a hydration medium, and the like. Delivery device 102 may be secured to patient 101 (e.g., to the body or clothing of patient 101) or may be at least partially implanted in the body of patient 101. In some embodiments, the delivery device 102 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of patient 101. The cannula and/or a needle may facilitate delivery of the therapeutic substance to a tissue layer, vein, interstitial fluid, or body cavity of patient 101. During operation, the actuator, in response to a signal (e.g., a command signal), may drive the delivery mechanism, thereby causing the therapeutic substance to move from the reservoir, through the cannula, and into the body of patient 101.

The components of delivery device 102 described above are merely provided as an example. Delivery device 102 may include other components, such as, without limitation, a power supply, a communication transceiver, one or more processors or other computing resources, memory devices, and/or user interfaces (e.g., buttons, keys, display, etc.), among other things. In some implementations, delivery device 102 may host an App (e.g., an insulin calculator) that may calculate the desired amount of therapeutic substance to be delivered to patient 101. Persons skilled in the art will recognize various implementations of delivery device 102 and the components of such implementations. All such implementations and components are contemplated to be within the scope of the present disclosure.

Monitoring device 104 may be configured to detect a physiological condition (e.g., a glucose concentration level) of patient 101 and may also be configured to detect other things. Monitoring device 104 may be secured to the body of patient 101 (e.g., to the skin of patient 101 via an adhesive) and/or may be at least partially implanted into the body of patient 101. Depending on the particular location or configuration, monitoring device 104 may be in contact with biological matter (e.g., interstitial fluid and/or blood) of patient 101.

Monitoring device 104 may include one or more sensors (not shown), such as, without limitation, electrochemical sensors, electrical sensors, and/or optical sensors. As persons skilled in the art will understand, an electrochemical sensor may be configured to respond to the interaction or binding of a biological marker to electrodes by generating an electrical signal based on, for example, a potential, conductance, current, and/or impedance of an electrical path through the electrodes. The electrodes may include a material selected to interact with a particular biomarker, such as glucose. The potential, current, conductance, and/or impedance may correlate with a concentration of the particular biomarker. In one example, the electrochemical sensor may include a glucose limiting membrane (GLM) that limits the amount of glucose and oxygen delivered to a glucose oxidase (GOx) layer of a working electrode of the sensor to ensure that the reactions are glucose limited. The GOx layer or another active enzyme layer on the working electrode of the sensor may break down glucose and oxygen into gluconic acid and hydrogen peroxide. The generated peroxide molecules may interact with the working electrode to break down hydrogen peroxide into two hydrogen ions, oxygen, and two electrons at the surface of the working electrode, when a voltage signal is supplied to the working electrode. The electrical charges may be forced to move between electrodes (e.g., between the working electrode and counter electrode), thereby generating a sensor current signal (Isig) that can be measured by sensor electronics. Other signals such as the counter voltage (Vcntr, the voltage potential difference between the counter electrode and the working electrode), electrochemical impedance spectroscopy (EIS) at different frequencies, and the like, may also be measured. The signals measured using the sensor, including the Isig, Vcntr, and EIS, may be processed (e.g., filtered or transformed) to generate some other signals or parameters, such as filtered Isig signals, real and imaginary impedance at various frequencies, and the like. These signals and/or the processed parameters may be used in one or more sensor glucose (SG) models (e.g., machine learning models or mathematical models) to determine SG values that may be estimations of the blood glucose (BG) levels of the patient.

As persons skilled in the art would understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of patient 101. In some embodiments, the electrical biosignal may include portions of a potential change produced by the heart of patient 101 over time (e.g., recorded as an electrocardiogram) that may be indicative of a glucose level of patient 101. An optical sensor may be configured to, for example, respond to the interaction or binding of a biological marker to a substrate by generating an electrical signal based on change in luminance of the substrate. In one example, the substrate may include a material selected to fluoresce in response to contact with a selected biomarker, such as glucose. The fluorescence may be proportional to a concentration of the selected biomarker.

In some embodiments, monitoring device 104 may include other types of sensors that may be worn, carried, or coupled to patient 101 to measure activity of patient 101 that may influence the glucose levels or glycemic response of patient 101. As an example, the sensors may include an acceleration sensor configured to detect an acceleration of patient 101 or a portion of the patient 101, such as the person's hands or feet, the position changes of which may be associated with an activity of patient 101. For example, the acceleration or movement (or lack thereof) of the body portion of patient 101 may be indicative of exercise, sleep, or food/beverage consumption activity of patient 101, which may influence the glycemic response of patient 101. In some embodiments, the sensors may measure heart rate and/or body temperature, which may indicate an amount of physical exertion experienced by patient 101. In some embodiments, the sensors may include a Global Positioning System (GPS) receiver which may detect GPS signals to determine a location of patient 101.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor may be referred to herein as a "sensor signal." As used herein, the term "sensed data" may mean and include the information represented by a sensor signal or by a pre-processed sensor signal. In some embodiments, sensed data may include glucose levels of patient 101, acceleration of a part of patient 101, heart rate of patient 101, temperature of patient 101, and/or geolocation (e.g., GPS location) of patient 101, among other things. Monitoring device 104 may communicate sensed data to delivery device 102 via communication link 112 and/or to computing device 106 via communication link 114. Use of sensed data by delivery device 102 and/or by computing device 106 is described in more detail below.

In some embodiments, monitoring device 104 may include components and/or circuitry configured to pre-process sensor signals. Pre-processing may include, for example, amplification, filtering, attenuation, scaling, isolation, normalization, transformation, sampling, and/or analog-to-digital conversion, among other things. In some embodiments, monitoring device 104 may host an App for process the sensor signals. In some embodiments, monitoring device 104 may include a wired or wireless transceiver as described above for transmitting the sensor signals or receiving commands or instructions. Persons skilled in the art will recognize various implementations for such pre-processing, including, without limitation, implementations using processors, controllers, integrated circuits, application specific integrated circuits (ASICs), hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, monitoring device 104 may not perform pre-processing.

Computing device 106 may provide processing capabilities and may be implemented in various ways. In some embodiments, computing device 106 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of delivery device 102. In some embodiments, computing device 106 may be processing circuitry that may be integrated with another device, such as delivery device 102. In some embodiments, computing device 106 may be secured to patient 101 (e.g., to the body or clothing of patient 101), may be at least partially implanted into the body of patient 101, and/or may be held by patient 101.

For each of the embodiments of computing device 106, computing device 106 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Some aspects of delivery device 102, monitoring device 104, and computing device 106 have been described above. One or more of devices 102-106 may include a user interface (not shown) that presents information to patient 101 and/or receives information from patient 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where computing device 106 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify patient 101 of sensed data (e.g., glucose level) and/or insulin delivery data (e.g., rates of historic, current, or future insulin delivery) and may present alerts to patient 101. In some embodiments, a user interface may receive inputs from patient 101, which may include, for example, a requested change in insulin delivery setting and/or a meal indication, among other things. The descriptions and embodiments above regarding user interfaces are merely provided as examples, and other types and other uses of user interfaces are contemplated to be within the scope of the present disclosure.

In one specific example, the communications between devices 102-106 and cooperation between devices 102-106 may be used for insulin delivery. As depicted in FIG. 1 and as described above, devices 102-106 may communicate with each other via communication links 112-116. In some embodiments, computing device 106 may control operations of delivery device 102 and/or monitoring device 104. For example, computing device 106 may generate one or more signals (e.g., a command signal) that cause delivery device 102 to deliver insulin to patient 101, for example, as a basal dosage and/or a bolus dosage. In some embodiments, computing device 106 may receive data associated with insulin delivery (e.g., insulin delivery data) from delivery device 102 and/or receive sensed data (e.g., glucose levels) from monitoring device 104, and may perform computations based on the insulin delivery data, the sensed data, and/or other data to control delivery device 102. Insulin delivery data may include, but is not limited to, the type of insulin being delivered, historical insulin delivery rates and/or amounts, current insulin delivery rate and/or amount, insulin delivery time, and/or user inputs affecting insulin delivery. As persons skilled in the art will understand, in a closed-loop operating mode, computing device 106 may communicate dosage commands to delivery device 102 based on, for example, a difference between a current glucose level in the body of patient 101 (e.g., received from monitoring device 104) and a target glucose level (e.g., determined by computing device 106 or set on delivery device 102). The dosage commands may indicate an amount of insulin to be delivered and/or a rate (or time) of insulin delivery, and may regulate the current glucose level toward the target glucose level.

Remote/cloud computing system 108 may be any proprietary remote/cloud computing system or a commercial cloud computing system including one or more server computing devices. Remote/cloud computing system 108 may provide alternative or additional computing resources as needed when the computing resources of a client computing device (e.g., computing device 106) are not sufficient. Computing device 106 and remote/cloud computing system 108 may communicate with each other through a communication link 118, which may traverse one or more communication networks (not shown). The communication networks may include, for example, an Ethernet network, a Wi-Fi network, a cellular network, a satellite network, an intranet, an extranet, the Internet, and/or the Internet backbone, among other types of networks. Persons skilled in the art will recognize implementations for remote/cloud computing system 108 and how to interface with such systems through various types of networks. For example, remote/cloud computing system 108 may include an array of processing circuitry and may execute machine-readable instructions. Such implementations, interfaces, and networks are contemplated to be within the scope of the present disclosure.

In some embodiments, remote/cloud computing system 108 may make a therapy determination (e.g., an insulin amount or adjusted insulin amount), and may communicate the therapy to delivery device 102 via computing device 106. In some embodiments, computing device 106 may make the therapy determination and communicate it to delivery device 102. In some embodiments, monitoring device 104 may make the therapy determination and communicate it to delivery device 102 either directly or through an intermediary such as computing device 106.

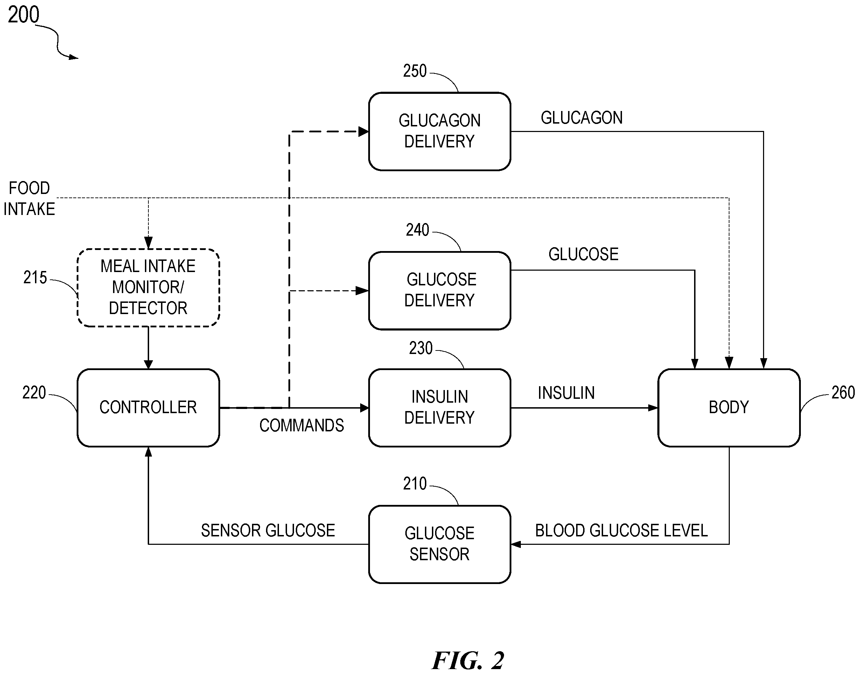

FIG. 2 is a block diagram of an example of a glucose control system 200 according to certain embodiments. In the illustrated example, glucose control system 200 may include a glucose sensor subsystem 210, a controller 220, an insulin delivery subsystem 230, a glucose delivery subsystem 240, and a glucagon delivery subsystem 250. Glucose sensor subsystem 210 may generate sensor glucose (SG) signals (e.g., SG levels) that may be the estimations of blood glucose levels in a body 260, and may provide the SG signals to controller 220. Controller 220 may receive the SG signals and generate commands to insulin delivery subsystem 230, and, in some implementations, glucose delivery subsystem 240 and/or glucagon delivery subsystem 250. Insulin delivery subsystem 230 may receive commands from controller 220 and deliver insulin to body 260 according to the commands. In some embodiments, glucose delivery subsystem 240 may receive commands from controller 220 and provide glucose into body 260 according to the commands. In some embodiments, glucagon delivery subsystem 250 may receive commands from controller 220 and deliver glucagon into body 260 according to the commands.

In some implementations, glucose sensor subsystem 210 may include a glucose sensor, sensor electronics configured to generate SG signals, a sensor communication system configured to send the SG signals to controller 220, and a housing for the sensor electronics and the sensor communication system. The glucose sensor may measure blood glucose levels, for example, directly from a blood stream, or indirectly via interstitial fluid using a subcutaneous sensor as described in more detail below.

Controller 220 may include electrical components and software to generate commands for insulin delivery subsystem 230, glucose delivery subsystem 240, and/or glucagon delivery subsystem 250. Controller 220 may include a controller communication system to receive the sensor signal and provide the commands to insulin delivery subsystem 230, glucose delivery subsystem 240, and/or glucagon delivery subsystem 250. In some implementations, controller 220 may implement a glucose calculator. In some implementations, controller 220 may include a user interface and/or operator interface (not shown) comprising a data input device and/or a data output device. Such a data output device may, for example, generate signals to initiate an alarm and/or include a display or printer for showing status of controller 220 and/or a patient's vital indicators. Such a data input device may include dials, buttons, pointing devices, manual switches, alphanumeric keys, a touch-sensitive display, combinations thereof, and/or the like for receiving user and/or operator inputs. Such a data input device may be used for scheduling and/or initiating insulin bolus injections for meals, for example. It should be understood, however, that these are merely examples of input and output devices that may be a part of an operator and/or user interface and that claimed subject matter is not limited in these respects.

Insulin delivery subsystem 230 may include, for example, an infusion device and/or an infusion tube to infuse insulin into body 260. Similarly, glucose delivery subsystem 240 may include, for example, an infusion device and/or an infusion tube to infuse glucose into body 260. Likewise, glucagon delivery subsystem 250 may include, for example, an infusion device and/or an infusion tube to infuse glucagon into body 260. In some embodiments, the insulin, glucagon, and/or glucose may be infused into body 260 using a shared delivery system and/or infusion tube. In some embodiments, the insulin, glucagon, and/or glucose may be infused using an intravenous system for providing fluids to a patient (e.g., in a hospital or other medical environment). It should be understood, however, that certain example embodiments may include an insulin delivery subsystem 230 without a glucagon delivery subsystem 250 and/or without a glucose delivery subsystem 240. In some embodiments, each of insulin delivery subsystem 230, glucose delivery subsystem 240, and glucagon delivery subsystem 250 may include infusion electrical components to activate an infusion motor according to the commands from controller 220, an infusion communication system to receive commands from controller 220, and a delivery subsystem housing.

In some embodiments, controller 220 may be housed in a delivery subsystem housing, and an infusion communication system may comprise an electrical trace or a wire that carries the commands from controller 220 to the delivery subsystem. In some embodiments, controller 220 may be housed in a sensor system housing, and a sensor communication system may comprise an electrical trace or a wire that carries the sensor signal from sensor electrical components to controller electrical components. In some embodiments, controller 220 may have its own housing or may be included in a supplemental device. In some embodiments, controller 220 may be co-located with a delivery subsystem and a sensor system within a single housing. In some embodiments, a sensor, a controller, and/or infusion communication systems may utilize a cable; a wire; a fiber optic line; RF, IR, or ultrasonic transmitters and receivers; combinations thereof; and/or the like instead of electrical traces, just to name a few examples.

In some embodiments, glucose control system 200 may also include a meal intake monitoring subsystem 215. Meal intake monitoring subsystem 215 may be used to log the amount of user food intake, or may automatically detect the amount of user food intake. For example, in some implementations, the user may enter the food items and/or the estimated amount of carbohydrates in a meal at the meal time. In some implementations, meal intake monitoring subsystem 215 may include sensors (e.g., cameras or accelerators) that may automatically detect a meal event, the food items in a meal, and/or the estimated amount of carbohydrates in the meal. The estimated amount of carbohydrates in the meal may be sent to controller 220, which may determine an appropriate amount of meal bolus and generate a command for insulin delivery subsystem 230 to deliver the meal bolus. In some implementations, meal intake monitoring subsystem 215 may not be used in glucose control system 200, and the dosage for the meal bolus may be determined based on the measured glucose level.

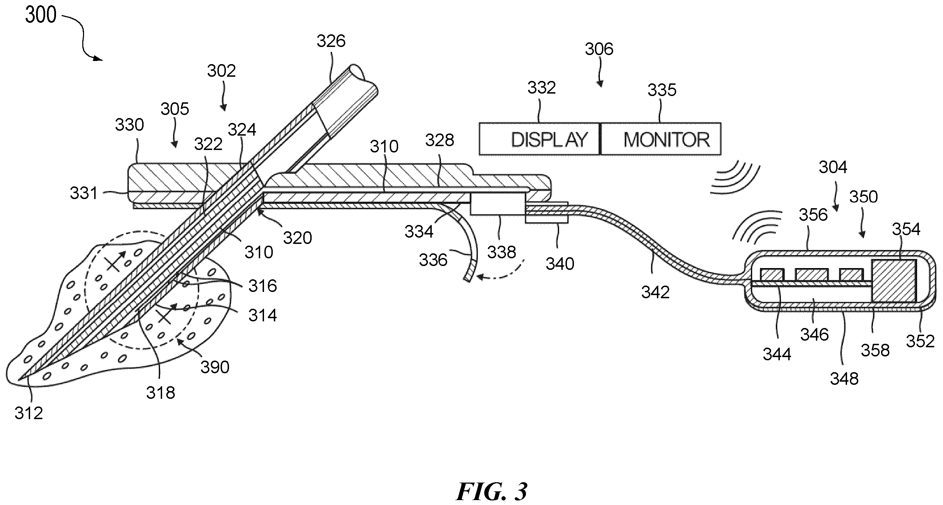

**FIG. 3** is a perspective view of an example of a CGM system 300 according to certain embodiments. In the illustrated example, CGM system 300 may include a subcutaneous sensor set 302 provided for subcutaneous placement of an active portion of a flexible sensor 310, or the like, at a selected site in the body of a user. The subcutaneous or percutaneous portion of sensor set 302 includes a hollow, slotted insertion needle 326 having a sharpened tip 312, and a cannula 322. Sensor set 302 may facilitate accurate placement of flexible sensor 310 into the body of the user. Inside cannula 322 is a glucose sensing portion 318 of flexible sensor 310. Glucose sensing portion 318 includes one or more glucose sensor electrodes 316 that may be exposed to the user's bodily fluids 390, for example, through a window 314 formed in the cannula 322. Sensor electrodes 316 may include, for example, a counter electrode, a reference electrode, and one or more working electrodes.

The proximal part of flexible sensor 310 may be mounted in a mounting base 305 adapted for placement onto the skin of the user. In some embodiments, mounting base 305 can be a pad having an underside surface coated with a suitable pressure sensitive adhesive layer 334, with a peel-off paper strip 336 normally provided to cover and protect the adhesive layer 334, until sensor set 302 is ready for use. Mounting base 305 may include an upper layer 330 and a lower layer 331, with a connection portion 328 of flexible sensor 310 being sandwiched between upper layer 330 and lower layer 331. Connection portion 328 may include a forward section joined to glucose sensing portion 318 of flexible sensor 310, which may be folded angularly to extend downwardly through a bore 320 formed in lower layer 331 of mounting base 305. Optionally, adhesive layer 334 (or another portion of the apparatus in contact with *in vivo* tissue) may include an anti-inflammatory agent to reduce an inflammatory response and/or antibacterial agent to reduce the chance of infection. Insertion needle 326 may be adapted for slide-fit reception through a needle port 324 formed in upper layer 330 of mounting base 305 and through lower bore 320 in the lower layer 331 of mounting base 305. In some embodiments, insertion needle 326 may be withdrawn after insertion to leave glucose sensing portion 318 (including glucose sensor electrodes 316) and/or cannula 322 in place at the selected insertion site.

Flexible sensor 310 of subcutaneous sensor set 302 may be connected to a sensor electronics device 304, which may be referred to as a transmitter. For example, mounting base 305 may be designed so that glucose sensing portion 318 may be joined to a connection portion 328 that terminates in conductive contact pads, or the like. Connection portion 328 and the contact pads may be adapted for an electrical connection to sensor electronics device 304 for determining the glucose level of the user in response to signals from sensor electrodes 316. Connection portion 328 may be connected electrically to sensor electronics device 304 by a connector block 338. Sensor electronics device 304 may include, for example, a housing 350 that supports a printed circuit board 344, batteries 354, and an antenna 346. In some embodiments, housing 350 may include an upper case 356 and a lower case 352 that are sealed by, for example, ultrasonic welding, to form a waterproof (or resistant) seal to permit cleaning by immersion (or swabbing) with water, cleaners, alcohol or the like. In some embodiments, upper case 356 and lower case 352 may be formed from a medical grade plastic. In alternative embodiments, upper case 356 and lower case 352 may be connected together by other methods, such as snap fits, sealing rings, RTV (silicone sealant) and bonded together, or the like, or formed from other materials, such as metal, composites, ceramics, or the like. In other embodiments, the assembly may be potted in epoxy or other moldable materials that is compatible with the electronics and reasonably moisture resistant. In some embodiments, sensor electronics device 304 may be connected to flexible sensor 310 through connector block 338 and a connector 340, and may be mounted onto mounting base 305. In some embodiments, sensor electronics device 304 may be connected to flexible sensor 310 through connector block 338, connector 340, and a cable 342. In some embodiments, lower case 352 may have a bottom surface coated with a suitable pressure sensitive adhesive layer 358, with a peel-off paper strip 348 normally provided to cover and protect the adhesive layer 358, until sensor electronics device 304 is ready for use.

Batteries 354 may include chargeable or non-chargeable batteries. In some embodiments, batteries 354 may include three series silver oxide battery cells. In other examples, different battery chemistries may be utilized, such as lithium based chemistries, alkaline batteries, nickel metalhydride, or the like, and a different number of batteries may be used. Batteries 354 may provide power to sensor set 302 via sensor electronics device 304 through, for example, connector block 338, connector 340, and/or cable 342.

Sensor electronics device 304 may include a processor, a transceiver unit, signal processing/conditioning circuits, and memory devices on printed circuit board 344. The processor may be configured to receive current and/or voltage signals from sensor electrodes 316 of flexible sensor 310. Sensor electrodes 316 may generate sensor signals indicative of a concentration of analyte (e.g., glucose) being measured. In one example, the sensor signals may include a current measured at a working electrode that may include a glucose oxidase (GOx) enzyme for catalyzing a reaction with glucose. The sensor signals may also include voltage signals measured at a counter electrode of sensor electrodes 316, or between a working electrode and a counter electrode (or reference electrode). In some examples, other electrical parameters, such as capacitance, resistance, impedance, and the like, may also be measured using sensor electrodes 316 inserted into the body of the user.

An example of the impedance parameters measured using sensor electrodes 316 may include electrochemical impedance spectroscopy (EIS) values. EIS may provide additional information in the form of sensor impedance and impedance-related parameters at different frequencies. Moreover, for certain ranges of frequencies, impedance and/or impedance-related data may be substantially glucose independent. Such glucose independence may enable the use of a variety of EIS-based markers or indicators for not only producing a robust, highly reliable sensor glucose value (e.g., through fusion methodologies), but also assessing the condition, health, age, and efficiency of individual electrode(s) of the overall sensor substantially independent of the glucose-dependent current signal. In one example, the analysis of the glucose-independent impedance data may provide information about the efficiency of a glucose sensor with respect to how quickly it hydrates and is ready for data acquisition. In addition, glucose-independent impedance data (using, e.g., values for 1 kHz real impedance) may provide information on potential occlusion(s) that may exist on a sensor membrane surface, where the occlusion(s) may temporarily block passage of glucose into the sensor and thus cause the signal to dip. Furthermore, glucose-independent impedance data may provide information on loss of sensor sensitivity during extended wear, for example, using values of phase angle and/or imaginary impedance at 1 kHz and higher frequencies.

The processor of sensor electronics device 304 may receive the sensor signals and calibrate the sensor signals using a calibration function that may be determined, for example, using reference values. In some embodiments, parameters (e.g., calibration factor or sensor sensitivity) of the calibration function may be determined (e.g., using calibration data) or pre-determined and stored in the memory for use by the processor. For example, in some embodiments, the processor may implement or otherwise execute a calibration application module that calculates or otherwise determines calibration parameters based on the measurement values and references values (e.g., blood glucose values measured using, for example, a fingerstick blood glucose meter). Based on the sensor signals and the determined calibration function, the processor may estimate the blood glucose level of the user. In some embodiments, the processor may store the glucose measurements in the memory. The sensor measurement results may be displayed on a display of sensor electronics device 304 or may be sent to a receiver 306 for display.

The memory of sensor electronics device 304 may be any type of memory device and may be configured to store raw sensor signals from flexible sensor 310 and/or glucose measurements (e.g., SG values) produced by the processor, calibration parameters used to determine glucose measurements from sensor signals, or other data used and/or produced by the processor. The memory may further store software and/or firmware that is executable by the processor. In some embodiments, the memory may further include configuration information for configuring the operation of sensor electronics device 304.

The signal processing/conditioning circuits of sensor electronics device 304 may include one or more amplifiers, filters, current to voltage converters, integrators, analog-to-digital converters, potentiostat, and the like. The signal processing/conditioning circuits may filter the signals, apply a constant reference voltage level, amplify current or voltage signals, sample and convert analog current or voltage signals into digital data values, and the like.

The transceiver unit of sensor electronics device 304 may include a wireless transceiver that may send the glucose measurements determined by the processor and stored in the memory to receiver 306 using a suitable wireless communication protocol, such as Bluetooth (e.g., Bluetooth low energy (BLE)), Wi-Fi, WiMax, and the like. For example, the transceiver unit may send radio signals indicative of the glucose level at regular time periods (e.g., every 5 minutes) to provide real-time sensor glucose (SG) values. The transceiver unit may also receive data, such as configuration data and calibration data (e.g., reference glucose values), from receiver 306. In some embodiments, the transceiver unit of sensor electronics device 304 may send data to receiver 306 through a cable or wire.

Receiver 306 may include a monitor 335 for monitoring physiological characteristics readings and/or generating alert signals, and a display 332 for displaying physiological characteristics readings and/or alert signals. For example, SG values/graphs may be displayed on display 332 of receiver 306 so that a user can monitor the blood glucose level and administer insulin using an insulin pump. In various embodiments, receiver 306 may be implemented in a desktop computer, a pager, a television including communications capabilities, a laptop computer, a server, a network computer, a personal digital assistant (PDA), a smart phone, an infusion pump including a display, a glucose sensor including a display, a combined infusion pump/glucose sensor, and the like.

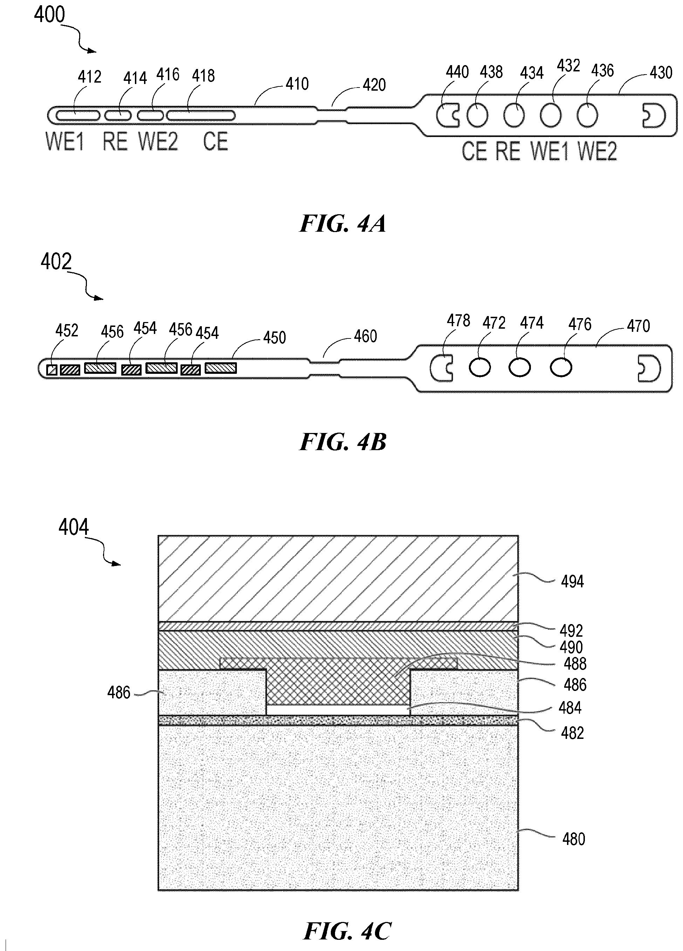

FIG. 4A illustrates an example of a glucose sensor 400 according to certain embodiments. Glucose sensor 400 may be an example of flexible sensor 310 described above. In the illustrated example, glucose sensor 400 may include a sensing portion 410 that includes one or more electrodes, such as a first working electrode (WE1) 412, a reference electrode (RE) 414, a second working electrode (WE2) 416, and a counter electrode (CE) 418, where the electrodes may be electrically isolated by an isolation layer. Sensing portion 410 may be an in vivo portion (also referred to as a distal end) that may be inserted into the subcutaneous layer of a user using a hollow needle, such as insertion needle 326. Glucose sensor 400 may include a bending region 420, which may be bent by, for example, up to about 90°, before or after insertion. Glucose sensor 400 may also include a contact pad region 430, which may be an ex vivo portion of glucose sensor 400. As illustrated, contact pad region 430 may include one or more contact pads electrically connected to the one or more electrodes in sensing portion 410 by conductive traces (not shown). The contact pads may include, for example, a contact pad 438 connected to counter electrode 418, a contact pad 434 connected to reference electrode 414, and two contact pads 432 and 436 connected to first working electrode 412 and second working electrode 416, respectively. The contact pads in contact pad region 430 of glucose sensor 400 may include, for example, a metal layer, and may be connected to one or more contact pads on a printed circuit board of a sensor electronics device. In the illustrated example, contact pad region 430 of glucose sensor 400 may also include a pair of guide holes 440 that may fit a pair of guide pins on the printed circuit board to align contact pads on contact pad region 430 with contact pads on the printed circuit board, such that voltage required for glucose sensing may be applied and sensor current proportional to interstitial glucose concentration may be measured by the sensor electronics on the printed circuit board.

**FIG. 4B** illustrates another example of a glucose sensor 402 according to certain embodiments. Glucose sensor 402 may be another example of flexible sensor 310 described above. In the illustrated example, glucose sensor 402 may include a sensing portion 450 that includes one or more electrodes, such as a reference electrode (RE) 452, one or more working electrode (WEs) 454, and one or more counter electrodes (CEs) 456, where the electrodes may be electrically isolated by an isolation layer. Sensing portion 450 may be an in vivo portion that may be inserted into the subcutaneous layer of a user using a hollow needle, such as insertion needle 326. Glucose sensor 402 may include a bending region 460, which may be bent by, for example, up to about 90°, before or after insertion. Glucose sensor 402 may also include a contact pad region 470, which may be an ex vivo portion of glucose sensor 402. As illustrated, contact pad region 470 may include one or more contact pads electrically connected to the one or more electrodes in sensing portion 450 by conductive traces (not shown). The contact pads may include, for example, a contact pad 472 connected to reference electrode 452, a contact pad 474 connected to working electrodes 454, and a contact pad 476 connected to counter electrodes 456. The contact pads in contact pad region 470 of glucose sensor 402 may include, for example, a metal layer, and may be connected to one or more contact pads on a printed circuit board of a sensor electronics device. In the illustrated example, contact pad region 470 of glucose sensor 402 may also include a pair of guide holes 478 that may fit a pair of guide pins on the printed circuit board to align contact pads on contact pad region 470 with contact pads on the printed circuit board, such that voltage required for glucose sensing may be applied and sensor current proportional to the interstitial glucose concentration may be measured by the sensor electronics on the printed circuit board.

**FIG. 4C** illustrates a cross-section of an example of a working electrode 404 of a glucose sensor according to certain embodiments. As illustrated, working electrode 404 may include a substrate or base layer 480 (e.g., including polyimide), which provides the foundation and rigidity of a flex substrate. An overlying plated metallic layer 482 (e.g., including chromium and/or gold) may be deposited on base layer 480. The chrome layer may promote adhesion between the polyimide and the gold layer, which enables the sensor to transfer charges when a bias voltage is applied. An electroplated layer 484 (e.g., including platinum) may be formed on metallic layer 482, e.g., in a trench formed in an insulating layer 486 (e.g., including polyimide). Insulating layer 486 may help to improve sensor integrity by providing electrical insulation between the working, counter, and reference electrodes, and may also serve as a supportive barrier for the electrodes and sensor flex chemistry. Electroplated layer 484 may be the active electrode for analyte (glucose) sensing, where glucose may be sensed at the working electrode through the oxidation of hydrogen peroxide to generate electrical charges. The working electrode may also be responsible for holding a bias potential $V_{set}$ applied by the sensor electronics. A glucose oxidase (GOx) layer 488 is formed on insulating layer 486, for example, by depositing a glucose oxidase solution (e.g., via slot coating) in the trench formed in insulating layer 486. A human serum albumin (HSA) layer 490 is formed overlying glucose oxidase layer 488. An adhesive promoter layer 492 may be used on HSA layer 490 to affix a glucose limiting membrane (GLM) layer 494 on working electrode 404. It is noted that FIG. 4C depicts a simplified representation of an example of a working electrode, and practical embodiments may include any number of additional and/or alternative layers (e.g., a high-density amine (HDA) layer, etc.). Accordingly, the subject matter described herein is not intended to be limited to the embodiment depicted in FIG. 4C. A reference electrode or counter electrode may have structures similar to the structure shown in FIG. 4C but may have some modification. For example, in some embodiments, a reference electrode may include an Ag layer, rather than a platinum layer, and may not include a glucose oxidase layer.

Glucose oxidase in GOx layer 488 may include the active enzyme of the sensor. In the presence of oxygen, GOx may break down glucose into gluconic acid and hydrogen peroxide. HSA layer 490 may break down hydrogen peroxide into water. Adhesive promoter layer 492 may include, for example, 3-aminopropyltriethoxysilane (APTES), which is a compound that provides a better adhering surface between the HSA layer 490 and GLM layer 494. GLM layer 494 may limit the amount of glucose and oxygen delivered to GOx layer 488 of working electrode 404 to ensure that the reactions are glucose limited. GOx layer 488 or another active enzyme layer on working electrode 404 may break down glucose and oxygen into gluconic acid and hydrogen peroxide. The generated peroxide molecules may interact with working electrode 404 (e.g., biased at about 0.2 V to about 0.7 V) to break down hydrogen peroxide into two hydrogen ions, oxygen, and two electrons at the surface of working electrode 404. The electrical charges may be forced to move between the electrodes, thereby generating a sensor current signal (Isig) that can be measured by sensor electronics. For example, when a GOx enzyme is used as a catalytic agent in the glucose sensor, an electrical current may flow from the counter electrode to the working electrode if there is oxygen in the vicinity of the enzyme and the sensor electrodes. Other signals such as the counter voltage (Vcntr, e.g., the voltage potential difference between the counter electrode and the working electrode), electrochemical impedance spectroscopy (EIS) signals at different frequencies, and the like, may also be measured.

In some embodiments, measurements of the parameters of the electrodes of the electrochemical glucose sensors described above may be performed during and/or after the fabrication processes. For example, an imaginary impedance of the working electrode (and the counter electrode) may be measured after a platinum electroplating process to form electroplated layer 484. GOx solution activity measurements may be performed during or after the GOx solution preparation process prior to deposition, and the thickness of GOx layer 488 may be measured after the slot coating and selective patterning processes over working electrode 404. The HSA concentration may be measured during or after the

EP 4 545 002 A1

solution preparation process prior to coating HSA layer 490, and the thickness of GLM layer 494 may be measured after forming GLM layer 494. In some embodiments, these measurements may be performed using sacrificial or monitor instances of glucose sensors fabricated on a wafer. In some embodiments, additional fabrication process measurements such as surface roughness or other topographic measurements may be performed (e.g., via interferometry) for the electrodes during or after a fabrication process.

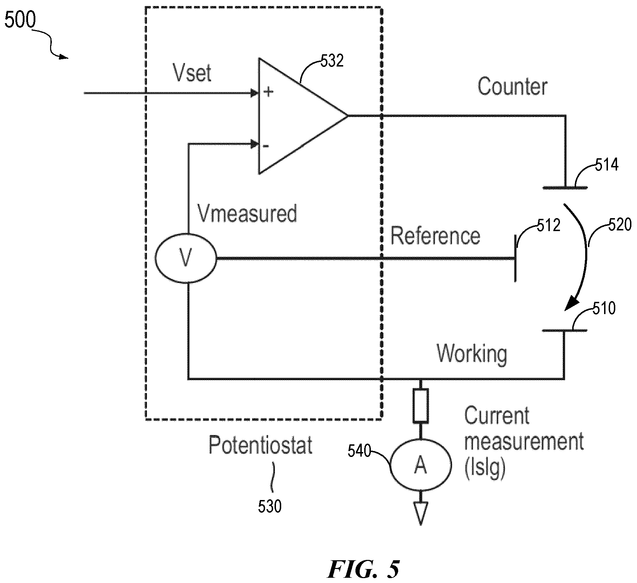

**FIG. 5** shows a schematic of an example of a glucose sensor 500 according to certain embodiments. Glucose sensor 500 may include a potentiostat 530 electrically connected to the electrodes of glucose sensor 500. As illustrated, potentiostat 530 may include an operational amplifier (op amp) 532 (or other servo-controlled device) that includes two inputs $V_{set}$ and $V_{measured}$. $V_{measured}$ may be the measured voltage between a reference electrode 512 and a working electrode 510 of a sensor flex as described above. $V_{set}$ may be the desired voltage across working electrode 510 and reference electrode 512. The output of op amp 532 may be connected to a counter electrode 514 to apply a voltage on counter electrode 514. Op amp 532 may utilize feedback through the sensor electrodes to attempt to maintain a desired voltage between reference electrode 512 and working electrode 510 by adjusting the voltage at counter electrode 514. Counter electrode 514 may balance the chemical reaction occurred at working electrode 510. A current 520 may flow between counter electrode 514 and working electrode 510 due to the electrochemical reaction between glucose and the sensor chemistry of working electrode 510 as described above. The current may be indicative of the glucose level in the interstitial fluid of the user and may be measured a current meter 540 as the sensor current (Isig) or may be output to processing circuits of sensor electronics for measurement. Other signals such as the counter voltage (Vcntr, e.g., the voltage potential difference between the counter electrode and the working electrode), electrochemical impedance spectroscopy (EIS) values at different frequencies, and the like, may also be measured using sensor electronics of the glucose sensor. Based on these raw sensor signals measured, the blood glucose level of the user may be determined using one or more models.

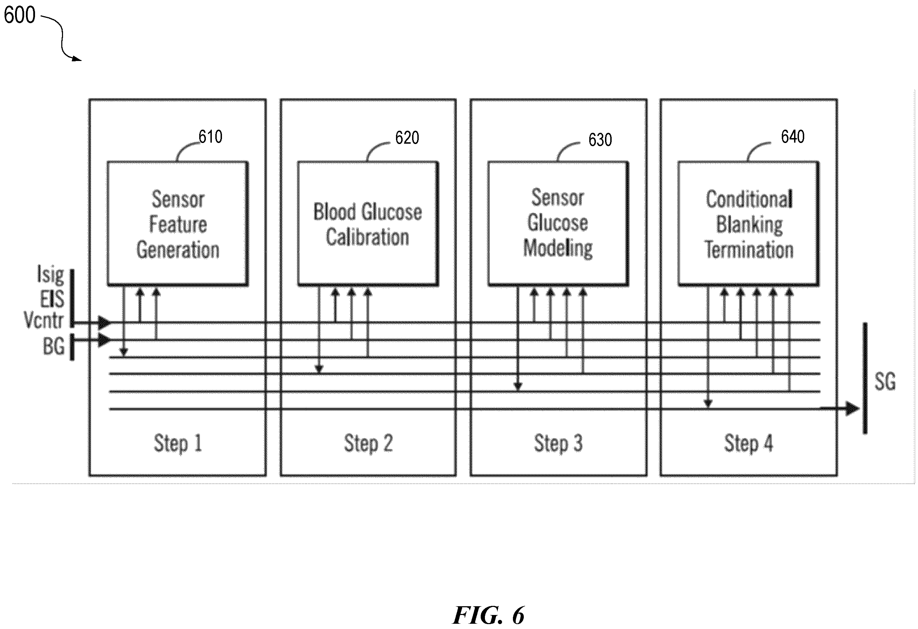

**FIG. 6** includes a flow diagram 600 illustrating an example of a method of determining sensor glucose values using input data from a glucose sensor, in accordance with certain embodiments. In the example illustrated in FIG. 6, the method may include sensor feature generation at block 610, optional blood glucose calibration at block 620, sensor glucose modeling at block 630, and optional conditional blanking and termination at block 640. In some embodiments, sensor feature generation at block 610 may use raw interstitial current signals, electrochemical impedance spectroscopy signals, and counter voltage signals to extract input features used by downstream SG models (e.g., machine learning models). The optional blood glucose calibration at block 620 may include adjusting the input sensor features from block 610 based on calibration parameters determined using reference blood glucose values. Sensor glucose modeling at block 630 may include applying machine learning techniques to the input features to convert the input features into sensor glucose values. Conditional blanking and termination at block 640 may include applying various tests and logic to determine, for example, when to stop displaying sensor output signals or terminate the sensor to reduce the probability of displaying noisy or erroneous information to the user or receiving device. In some embodiments, terminating the sensor may include ceasing transmission of sensor data from the sensor device. In some embodiments, input data (e.g., interstitially measured Isig, Vcntr, EIS, and/or BG values) may pass through blocks 610-640 and be transformed into SG values. Table 1 below shows an example of the inputs and outputs of each of the four blocks of flow diagram 600. In some embodiments, the processes of flow diagram 600 may be implemented using one or more machine learning models.

Table 1. Information Transfer in Flow Diagram 600

| Information | Block 610 | Block 620 | Block 630 | Block 640 |
|---|---|---|---|---|
| Isig, Vcntr, EIS, BG | Input | N/A | N/A | Input |
| Base and Derivative Sensor Features w/o Calibration | Output | Input | Input | Input |
| Base and Derivative Sensor Features after BG Calibration | N/A | Output | Input | Input |
| Initial Estimates of SG Values | N/A | N/A | Output | Input |
| Final Estimates of SG Values | N/A | N/A | N/A | Output |

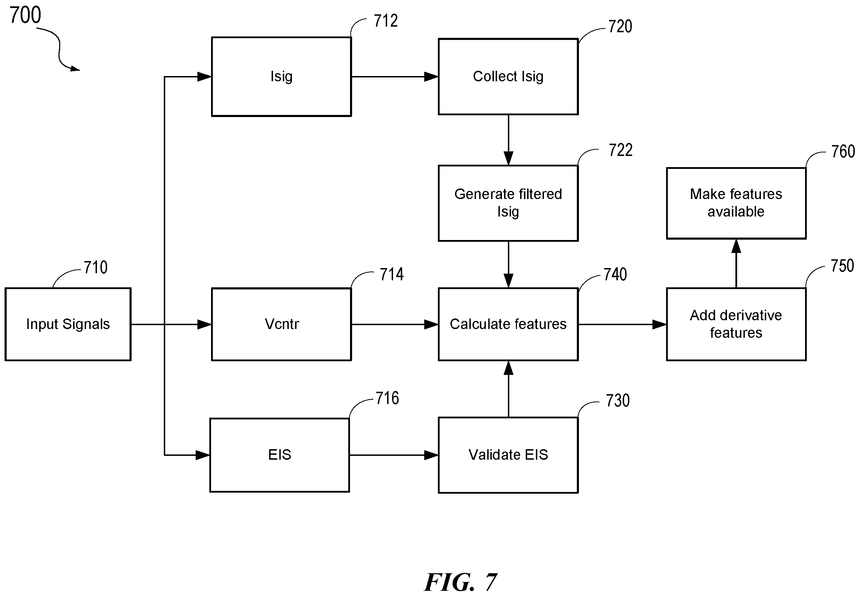

**FIG. 7** illustrates an example of a sensor feature generator 700 according to certain embodiments. Sensor feature generator 700 may be used to perform the operations of sensor feature generation at block 610 of FIG. 6. In the illustrated example, input signals 710 to sensor feature generator 700 may include Isig signals 712 (*e.g.,* at an interval about 1 minute), Vcntr signals 714 (*e.g.,* at an interval about 5 minutes), and EIS signals 716 (*e.g.,* at an interval about 15-30 minutes). Isig signals 712 may be collected over, for example, 5 or more minutes at block 720, and the collected Isig signals 712 may be used to generate filtered Isig signals 722 (e.g., over a 5-minute time window). For example, the most recent 8 minutes of Isig signals 712 may be filtered using, for example, a seventh order finite impulse response (FIR) filter to create a filtered Isig signal 722. In some implementations, any computed filtered Isig signal 722 that is invalid may be

14

discarded, for example, if a value of the recent 8 minutes of Isig signals 712 is larger than twice of the mean value of the 8 minutes of Isig signals 712 or lower than a half of the mean value of the 8 minutes of Isig signals 712. In some embodiments, artifact detection may be performed to identify large and small drops in Isig signals 712 or filtered Isig signal 722. EIS signals 716 may include measurements taken using signals of various frequencies, such as a set of frequencies between about 0.1 Hz and about 8 KHz. EIS signals 716 may be validated to generate a validated EIS signal 730. In some embodiments, validated EIS signal 730 may be converted to 5-minute sampling using zero-order hold. In some embodiments, the frequency of measuring the signals can change depending on the hardware design. For example, Vcntr may be measured at 1-minute and EIS may be measured more frequently, depending on factors such as battery life and memory size limitations.

The filtered Isig signal 722, validated EIS signal 730, and Vcntr signals 714 may be used to generate features 740, where the features for each time period (e.g., 5-minute time period) may be included in a respective data packet. Derivative features 750 may be added to the data packet based on features 740 in the current data packet and previous features 740 in previous data packets. In some embodiments, transformed features (not shown in FIG. 7) may also be generated by sensor feature generator 700 using, for example, a linear or non-linear projection of features 740 and/or derivative features 750. For example, Principal Component Analysis (PCA) or Autoencoder may be used to generate the transformed features. Final features 760 including features 740, derivative features 750, and/or the transformed features may include, for example, filtered Isig signal 722, previous filtered Isig signals, an accumulative average of Isig signals, a moving average of Isig signals, filtered Isig signals, Vcntr signal 714 of current data packet, Vcntr signals of previous data packets, sensor age, real and imaginary components of EIS signals at various frequencies, changes of the real and imaginary components of EIS signals at various frequencies, and the like. In some embodiments, sensor feature generator 700 may process the Isig signals, Vcntr signals, and EIS signals based on a different time interval (e.g., shorter or longer than 5 minute). A data packet including all or some of features 740, derivative features 750, and/or the transformed features may be used for optional blood glucose calibration at block 620, sensor glucose modeling at block 630, and optional conditional blanking and termination at block 640.

Due to manufacturing variances and aging of the glucose sensors, some glucose sensors may not provide accurate readings based on measured signals and derived signal features. As such, the glucose sensors may need to be occasionally or periodically calibrated in vitro and/or in vivo to improve the accuracy and/or reliability of the glucose sensors. As shown in the example of FIG. 6, the sensor signals may be calibrated at block 620 using calibration functions determined using known good blood glucose values, such as fingerstick measurements from blood glucose meters that measures the blood glucose level in the capillaries. For example, a calibration ratio CR (or a calibration factor) may be determined according to CR=BG/(Isig + offset). The input signals, such as Isig signals, may then be calibrated using the calibration ratio and/or the offset. In some embodiments, a weighted calibration ratio may be determined based on previous calibration ratios and time of the previous calibrations and applied to all subsequent data packets until next calibration is performed.

At block 630, sensor glucose values may be derived from the generated features using one or more SG models. The SG models may include, for example, functions, equations, machine learning models, or other translation models that may map the generated features, Isig signals, Vcntr signals, and/or EIS signals to a sensor glucose (SG) value indicative of an estimated blood glucose level. For example, the features generated at block 610 and/or block 620 may be input to the one or more SG models as a feature vector, and the SG value may be determined as the dot product of the feature vector and a coefficient vector with corresponding coefficients (e.g., weights). For example, an SG model may be represented by:

$$SG = f(Age, Isig, Vcntr, EIS).$$

In a simplified example, SG values may be calculated according to:

$$SG = \alpha \times Age + \beta \times Isig + \gamma \times Vcntr + \delta \times EIS + \Delta,$$

where $\alpha$, $\beta$, $\gamma$, and $\delta$ may be coefficients associated with the age, Isig, Vcntr, and EIS, and $\Delta$ is an offset value. In some embodiments, at least one of Isig, Vcntr, or EIS may include a vector, and at least one of $\beta$, $\gamma$, or $\delta$ may be a coefficient vector. In some embodiments, at least one of $\alpha$, $\beta$, $\gamma$, and/or $\delta$ may be a function of time (e.g., the sensor age).

As described above, sensor properties associated with a glucose sensor, such as the age (or time of use or wear time), battery level, calibration accuracy, sensing environment (e.g., oxygen level, temperature, moisture, and pH value, etc.), physiological dynamics, manufacturing variability, and other properties, may affect the enzymatic glucose level measurement results in complex ways that may be difficult to model. Therefore, many CGM sensors may have suboptimal accuracy and may exhibit large variance in the measurement results. The lower than desired performance of the CGM sensors may be at least partially attributed to the complexity of modeling the in vivo behavior of the CGM sensors, in addition to the sensor-to-sensor manufacturing variability, degradation of the sensor performance over time, and the like.

In many cases, a single SG model (e.g., a single equation) may not be able to accurately estimate the blood glucose level because various sensor properties may affect the glucose level measurement results in complex ways that may be difficult to characterize. In some embodiments, different SG models may be used for different input parameter ranges, such as different ranges of the age, Vcntr, or other parameters of the sensor measurement data. One model may be selected from the one or more models based on the ranges that the input parameters of the sensor measurement data fall in.

In some embodiments, the one or more models may include machine learning models. The machine learning models may include neural networks or other machine learning models. For example, the machine learning models may include neural networks that include a large number of nodes (neurons) arranged in one or more layers. Each node of a neural network may be connected with one or more other nodes of the neural network, where the connections may have associated coefficients or weights. In some embodiments, each individual node may implement a multiplication and accumulation function that combines the weighted values of all its inputs based on the associated weights. In some embodiments, each connection (or the node) may have a threshold function such that only signals that surpass the threshold may propagate to other nodes. The neural network may be trained or self-learning using training samples, rather than being explicitly programmed. In some embodiments, the neural network may include multiple layers each including multiple nodes, where input signals may traverse the multiple layers to generate a final output. In some embodiments, techniques such as back propagation techniques may be utilized to train the neural network, such as determining the coefficients or weight associated with the connections between nodes. Data for training the machine learning model may include, for example, sensor data (e.g., Isig, Vcntr, EIS, etc.) and corresponding known SG or BG values.

In some embodiments, the machine learning model may receive a multi-dimensional feature vector as inputs. For example, the inputs to the machine learning model may include features such as EIS features, Isig trending features, Isig rate of change features, previous Isig values, previous Vcntr values, and the like. Some examples of the input features and models used for determining SG values can be found in, for example, U.S. Pat. Appl. No. 16/773,422, entitled "METHODS, SYSTEMS, AND DEVICES FOR CONTINUOUS GLUCOSE MONITORING," which is herein incorporated by reference in its entirety. The outputs of the machine learning model may depend on combinations of the input features. In some embodiments, the one or more models may include a plurality (e.g., N) of models (e.g., functions, equations, machine learning models, etc.). The models may use the same or different input features and/or or may weigh the input features differently using different weights. In some embodiments, one or more models may be selected based on the input data and used to determine an SG value. For example, in some embodiments, outputs of two or more models selected from the plurality of models may be combined to generate a final SG value.

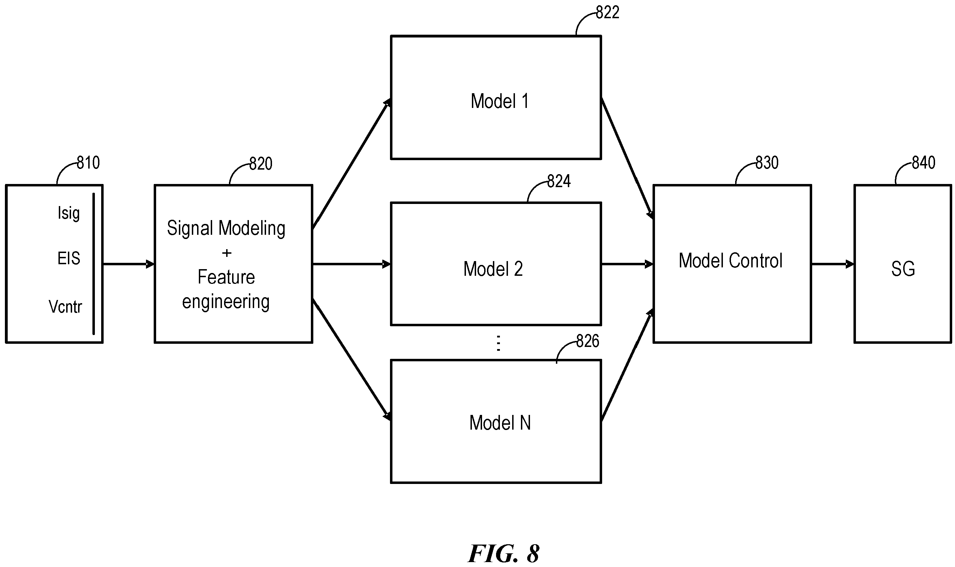

FIG. 8 illustrates an example of determining a sensor glucose value using one or more models according to certain embodiments. As shown in FIG. 8, inputs 810 to the one or more models may include Isig, EIS, Vcntr, or other input signals. In some embodiments, inputs 810 may additionally include prior modeling (e.g., trending) information. In the illustrated example, a signal modeling and feature engineering engine 820 may include N models, such as models 822, 824, ... , and 826, that may be trained or otherwise determined for various combinations of inputs or scenarios. As described above, the models may include machine learning models and may be trained to use different inputs or weigh the inputs differently. A model controller 830 may perform selections, averaging, ranking, weighting, or other processing of sensor glucose values generated by models 822, 824, ... , and 826. For example, model controller 830 may select a model (e.g., from models 822, 824, ... , and 826) based on inputs 810 or other information, such as age of the sensor, to determine an SG value. In some embodiments, model controller 830 may average the outputs of two or more models of models 822, 824, ... , and 826 to generate a final sensor glucose value 840. In some embodiments, model controller 830 may take the average, weighted average, median, maximum, minimum, and the like of the outputs of a subset of models 822, 824, ... , and 826. In some embodiments, model controller 830 may rank or weigh models 822, 824, ... , and 826, and may select the high ranking model(s). In some embodiments, a confidence score associated with the output of each of models 822, 824, ... , and 826 may be generated to indicate the confidence level (or probability) that the output is correct, and model controller 830 may adjust the model outputs to favor one or more models associated with the highest levels of confidence. The final SG value 840 may be displayed to the user or may be sent to an insulin pump or an insulin calculator. In some embodiments, a confidence score associated with SG value 840 may be generated to indicate the confidence level that the final SG value 840 is correct.

At block 640, conditional blanking and termination may be performed on the SG values generated by the SG models. For example, some SG values generated by the one or more SG models may be blanked and not sent or displayed to the user if the SG values are determined to be outliers or otherwise erroneous. In some embodiments, the conditional blanking and termination at block 640 may be performed by one or more models such as machine learning models used at block 630 or other machine learning models. For example, a machine learning model may classify the SG values as being outliers/not outliers, having large negative bias/large positive bias/nominal accuracy, having poor accuracy/ intermediate accuracy/good accuracy, and the like. The SG values may be blanked based on the outputs from the machine learning model. For example, if the output of the machine learning model indicates that an SG value is not an outlier (e.g., within a certain threshold range), the SG value may not be blanked. Otherwise, the SG value may be blanked. Similarly, if the output of the machine learning model indicates that an SG value has an intermediate, normal, or good accuracy, the SG value may not be blanked. Otherwise, the SG value may be blanked. In some embodiments, a sensor may be terminated (*e.g.,* stop calculating or transmitting any sensor data from that sensor and/or stop displaying any sensor data from that sensor) when, for example, a large number of outliers are generated using sensor measurement signals from the sensor.

In some implementations, to achieve the desired accuracy and reliability and reduce the impact of noise and other spurious signals, CGM sensors may need to be periodically or occasionally calibrated in vitro and/or in vivo to improve the accuracy and/or reliability of the glucose sensors. For example, the sensor data may need to be calibrated using a known good blood glucose value, often obtained via a so-called "fingerstick measurement" using a blood glucose meter that measures the blood glucose level in the capillaries. Performing such calibration measurements may increase the patient's burden and perceived complexity, and can be inconvenient, uncomfortable, or otherwise disfavored by patients. Moreover, ISF glucose measurements may lag behind the blood glucose measurements due to the time it takes glucose to diffuse from the capillary to the interstitial space, which may require certain signal processing (e.g., filtering) or other techniques to compensate for the physiological lag. Additionally, various factors can lead to transient changes in the sensor output, which may influence the accuracy of the calibration and the measurement. Degradation of sensor performance over time and manufacture variations between the sensors may further compound these challenges. Occasional or periodical calibration may improve the performance of the CGM sensors, but may not be able to adequately address all challenges related to the accuracy of the CGM sensors.

According to certain embodiments disclosed herein, different SG models may be used for different input parameter ranges (e.g., different subspaces of the input parameter space), such as different ranges of the age, Vcntr, Isig, EIS, or other sensor measurement data. Each SG model may be optimized for a respective region of the input parameter space (referred to as a regional SG model or regional model) using, for example, machine learning techniques, and thus may be able to more accurately characterize the relationship between the glucose level and the sensor measurement data within the respective region. For example, in some embodiments, the input parameter space may be partitioned based on the age of the sensor, the Vcntr, the sensor current Isig, another input parameter, or any combination thereof. In one example, the input parameter space may be divided based on two parameters, where one of the two parameters may include the sensor current Isig. In some examples, the input parameter space may be divided based on three or more parameters, where one of the three or more parameters may include the sensor current Isig. The input parameter space may be partitioned uniformly or nonuniformly into regions having regular or irregular shapes. The SG model for each region may be separately trained using a respective set of training data, or may be trained together using a same set of training data. In some embodiments, the SG models may use the same input parameters and have similar structures, but may have different coefficients or weights for a same input parameter. In some embodiments, the SG models for different regions of the input parameter space may use different input parameters and/or may include different types of model or different model structures.

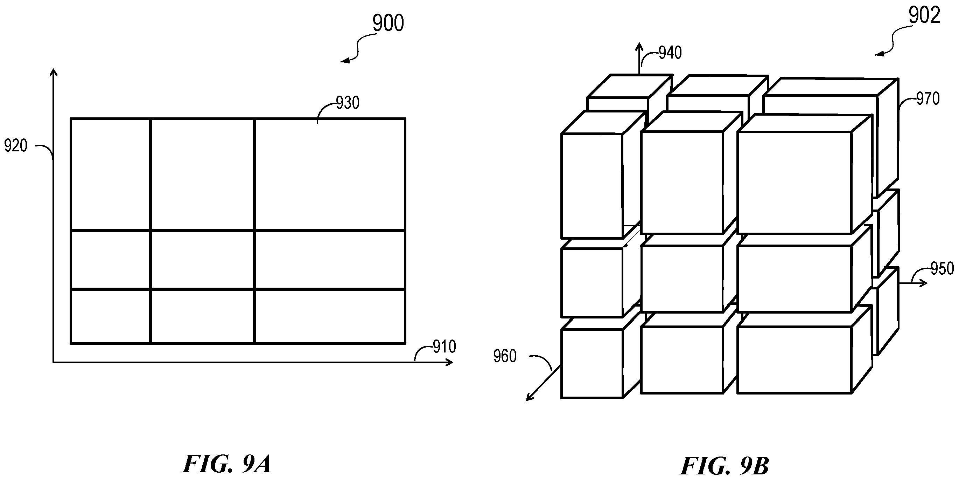

**FIG. 9A** shows an example of a partitioned model 900 including a set of regional models for modeling a relationship between SG values and sensor measurement data according to certain embodiments. In the illustrated example, the input parameter space may be partitioned based on ranges of two parameters 910 and 920, where one of the two parameters may include the sensor current Isig, and the other one of the two parameters may include, for example, sensor age, Vcntr, an in vivo characteristic of glucose sensors (e.g., sensitivity), another input parameter of the SG model, or an environmental property (e.g., moisture or temperature) of the glucose sensor. Each region 930 of the input parameter space may be associated with a respective regional SG model. Regions 930 may have the same or different sizes. Each region 930 may have a regular shape (e.g., rectangle) or an irregular shape, where a boundary between two adjacent regions may be a straight line or may be a curve. In one example, the boundaries between regions 930 may be determined using machine learning techniques such as support vector machines (SVMs).

**FIG. 9B** shows another example of a partitioned model 902 including a set of regional models for modeling a relationship between SG values and sensor measurement data according to certain embodiments. In the illustrated example, the input parameter space may be partitioned based on ranges of three or more parameters 940, 950, and 960, where one of the three or more parameters may include the sensor current Isig, and the other parameters of the three or more parameters may include, for example, the sensor age, Vcntr, an in vivo characteristic of glucose sensors (e.g., sensor sensitivity), another input parameter of the SG model, an environmental property (e.g., moisture or temperature) of the glucose sensor, or a combination thereof. A respective regional SG model can be generated for each region 970 of the input parameter space. Regions 970 may have the same or different sizes. Each region 970 may have a regular shape (e.g., a prism such as a rectangular prism) or an irregular shape, where a boundary between two adjacent regions may be a planar surface or may be a curved surface.

As shown in FIGS. 9A and 9B, the input parameter space may be partitioned into a plurality of regions that collectively forms the contiguous input parameter space. In some embodiments, to partition the input parameter space, each region of the input parameter space in which the sensor may have similar characteristics or may be modeled using a similar model may be identified. In some embodiments, the stability of sensor trends, regularity of sensor signal magnitudes, or other characteristics of the sensor signals may be identified and used to partition the input parameter space. In some embodiments, machine learning techniques, such as clustering, dimensionality reduction (e.g., principal component analysis (PCA)), SVMs, and the like, may be used to partition the input parameter space, including determining the parameters used for the partitioning and the boundaries of the partitions. In some embodiments, the partitioning and/or the models may be modified if the models used in one or more regions generate erroneous SG values (e.g., having a large Mean Absolute Relative Difference (MARD)), if the models for adjacent regions output significantly different SG values for similar input parameters, and the like.

In some embodiments, different models may be used in different regions 930 or 970 of the input parameter space. The models may include, for example, a linear regression model, a neural network, another machine learning model, a function, an equation, or another model. In some embodiments, a plurality of candidate models for each region of the input parameter space may be ranked based on the complexity, accuracy, and/or other metrics of the models, and one or more models may be selected for the region based on the ranking. This process may be performed for each region. In some embodiments, simpler models may be sufficient for certain regions while more complex models may be needed for other regions. Therefore, a combination of different types of models may be used. In some embodiments, some models may use the same input parameters and have similar structures but may have different coefficients or weights for a same input parameter.

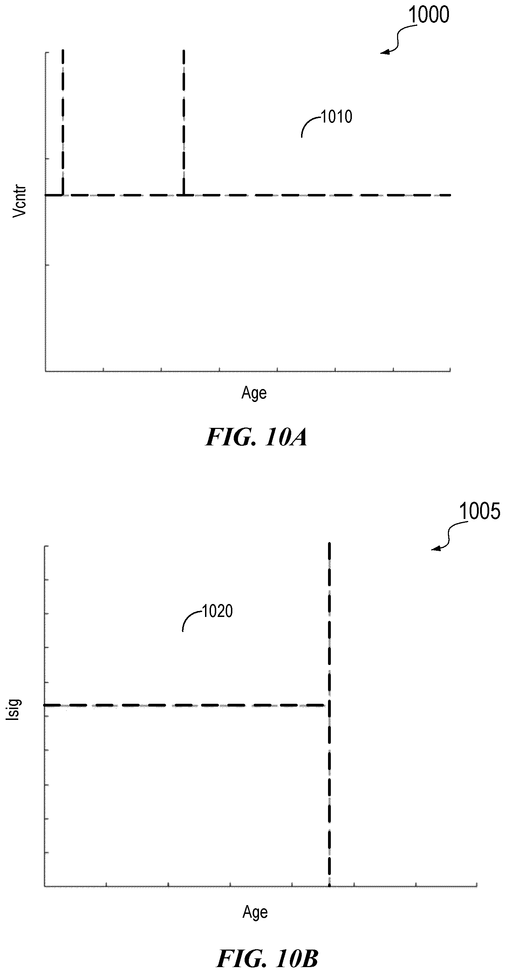

**FIG. 10A** shows an example of a scheme 1000 for partitioning the input parameter space into a plurality of regions 1010 associated with different respective regional SG models according to certain embodiments. In the illustrated example, the input parameter space may be partitioned based on ranges of the age of the glucose sensor and the Vcntr of the sensor measurement data. Each region 1010 of the input parameter space may have the same or different sizes and may be associated with a respective regional SG model. Each region 1010 may have a regular shape (e.g., rectangle) or an irregular shape, where a boundary between two adjacent regions may be a straight line or may be a curve, which may be determined by, for example, support vector machines or other machine learning techniques. In the illustrative example shown in FIG. 10A, a regional SG model may be used for input measurement data with Vcntr values greater than a certain threshold value (e.g., greater than about 0.7), and three different regional SG models may be used for input measurement data with Vcntr values lower than the threshold value and measured during different ages of the glucose sensor. It is noted that the particular partition scheme illustrated in FIG. 10B is for illustration purposes only and is not intended to limit the scope of this disclosure to the depicted example only.

**FIG. 10B** shows another an example of a scheme 1005 for partitioning the input parameter space into a plurality of regions 1020 associated with different respective regional SG models according to certain embodiments. In the illustrated example, the input parameter space may be partitioned based on ranges of the age of the glucose sensor and the sensor current Isig of the sensor measurement data. Each region 1020 of the input parameter space may have the same or different sizes and may be associated with a respective regional SG model. Each region 1020 may have a regular shape (e.g., rectangle) or an irregular shape, where a boundary between two adjacent regions may be a straight line or may be a curve, which may be determined by, for example, support vector machines or other machine learning techniques. In the illustrative example shown in FIG. 10B, a regional SG model may be used when the age of the glucose sensor is greater than a certain threshold value, and two different regional SG models may be used for input measurement data with Isig values in two different ranges and measured before the age of the glucose sensor reaches the threshold value. It is noted that the particular partition scheme illustrated in FIG. 10B is for illustration purposes only and is not intended to limit the scope of this disclosure to the depicted example only.

In some embodiments, various smoothing or blending techniques may be used in the regional models of adjacent regions, to avoid abrupt changes in output SG values at boundaries between adjacent regions caused by the different models used for the adjacent regions. For example, in some embodiments, the output SG value for input parameters near a boundary between two regions may be a weighted average of the output SG values from the two models for the two adjacent regions. The output SG value for input parameters at an area (e.g., a corner) adjacent to three or more adjacent regions may be a weighted average of the output SG values from the three or more models for the three or more adjacent regions. In other embodiments, other techniques for smoothing or blending the output SG values for input parameters at or near the region boundaries may be used.

According to certain embodiments, multiple sets of regional SG models may be generated and used to determine SG values that approximate the blood glucose levels of a user, where the different sets of regional SG models may be generated for different partitioning of the input parameter space (e.g., different combinations of input parameters ranges). The input parameters may include, for example, Isig, Vcntr, EIS, age, sensor sensitivity, etc. The input parameter space may be divided differently according to different partition schemes, for example, based on different combinations of input parameters, and/or by dividing the ranges of a same combination of input parameters differently. In some embodiments, the different partition schemes may use different numbers of input parameters. For example, one partition scheme may partition the input parameter space based on different ranges of two parameters, and another partition scheme may partition the input parameter space based on different ranges of three or more parameters. A set of regional SG models may be generated for regions of the input parameter space divided according to a respective partition scheme. One regional SG model may be selected from each set of regional SG models for a respective input parameter space partition scheme, based on the region in which the current input parameters fall. Multiple regional SG models selected from the multiple sets of regional SG models for the different partition schemes may be used to estimate the SG values independently, and the outputs of the selected multiple regional SG models may be combined (e.g., by averaging or weighted averaging) to determine a predicted SG value. In some embodiments, the manner in which the outputs of the selected multiple regional SG models are combined (e.g., the weights for the different outputs) may be determined using, for example, machine learning techniques.

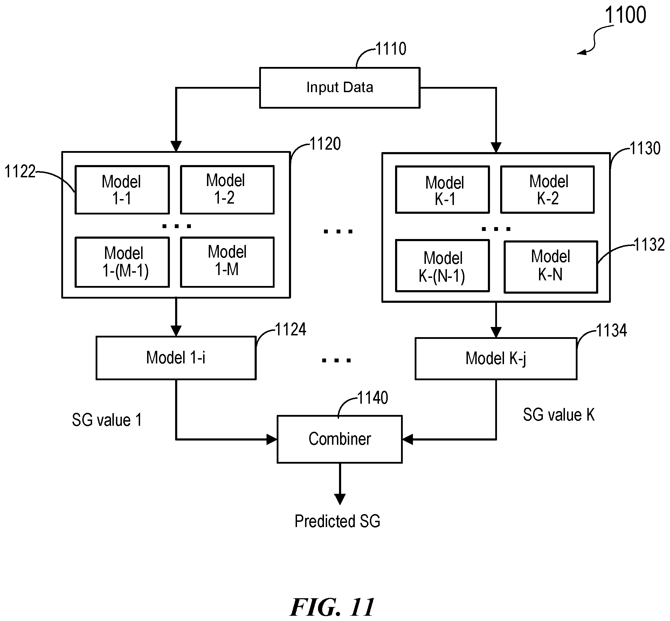

FIG. 11 illustrates an example of a process 1100 of estimating SG values using an ensemble of multiple sets of regional models according to certain embodiments. In the illustrated example, the input parameter space of the SG models for estimating SG values may be partitioned differently according to K (e.g., $\geq 2$) different partition schemes, where the K different partition schemes may use, for example, different combinations of input parameters, different ranges of same input parameters, different number of input parameters, or a combination thereof, and may partition the input parameter space into the same or different numbers of regions of the same or different sizes, shapes, and/or numbers of dimensions. A respective set of regional SG models may be determined for each partition scheme. For example, a first partition scheme 1120 may partition the input parameter space into M regions using one or more parameters (e.g., selected from Isig, age, Vcntr, and other input parameters), and a regional SG model 1122 (e.g., model 1-1, model 1-2, ... , model 1-(M-1), or model 1-M) may be determined for each respective region of the M regions. A Kth partition scheme 1130 may partition the input parameter space into N regions using one or more parameters (e.g., selected from Isig, age, Vcntr, and other input parameters), and a regional SG model 1132 (e.g., model K-1, model K-2, ... , model K-(N-1), or model K-N) may be determined for each respective region of the N regions.

When input data 1110 (e.g., sensor measurement data, its derivatives, and/or transformed sensor measurement data) is received, a controller or processor may, based on the values of the input parameters and the first partition scheme, select a first regional SG model 1-i (1124) from model 1-1, model 1-2, ... , model 1-(M-1), and model 1-M, and estimate a first SG value (SG value 1) using the first regional SG model 1-i (1124) and input data 1110. The controller or processor may also, based on the values of the input parameters and other partition schemes, select a respective regional SG model from each respective set of regional SG models for a respective partition scheme of the other partition schemes, and estimate a respective SG value using the selected regional SG model and input data 1110. For example, the controller or processor may select a Kth regional SG model K-j (1134) from model K-1, model K-2, ... , model K-(N-1), and model K-n, and estimate a Kth SG value (SG value K) using the Kth regional SG model K-j (1134) and input data 1110. In some embodiments, each regional SG model may also output an estimated probability, confidence level, or accuracy of the estimated SG value.

The outputs of the K selected regional SG models may be used in combination by a combiner 1140 to generated a predicted SG value for input data 1110. For example, combiner 1140 may determine an average or weighted average of the K SG values and use the average or weighted average as the predicted SG value for input data 1110. In one example, the weights for the K SG values may be determined based on the estimated probability or confidence level of each of the K SG values. In another example, the weights for the K SG values may be predetermined during a training or learning phase, where a weight may be determined for each respective set of regional SG models associated with a respective partition scheme.

Using the ensemble of multiple sets of regional SG models disclosed herein may more accurately model the sensor variance (e.g., sensitivity variations), and may significantly improve the performance (e.g., accuracy and consistency) of CGM sensors. Each regional model may be trained independently at a faster speed using a smaller set of training data, and may better characterize the response of the glucose sensor to analyte of different concentrations, such as the sensitivity and the nonlinearity of the sensitivity. Using certain parameters such as Isig as additional parameters for partitioning the input parameter space may also help to improve the performance of the SG models due to, for example, the improved granularity of partitioning the input parameter space and the relevancy of the additional parameters for modeling the response of the glucose sensor. Compared to using a one complex SG model or one set of regional SG models having a large number of models for a large number of regions partitioned using a large number of input parameters, using an ensemble of multiple sets of regional SG models for different partition schemes (e.g., using different input parameters for different partition schemes) may further improve the performance (e.g., accuracy and consistency) of CGM sensors due to, for example, the use of additional parameters to improve the granularity of the partitioning, and the use of additional partition schemes to include additional parameters or freedom for tuning and improving the SG models, without significantly increasing the overall complexity of the SG models and the difficulty of training the SG models.

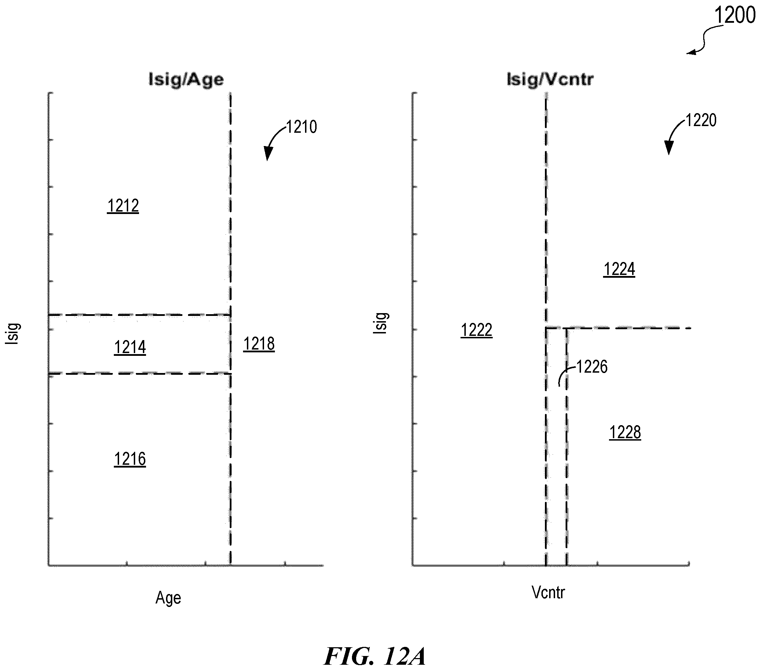

FIG. 12A illustrates an example of an ensemble model 1200 including multiple sets of regional SG models for estimating SG values based on sensor measurement data generated by a glucose sensor according to certain embodiments. It is noted that particular partition schemes in the ensemble model illustrated in FIG. 12A is for illustration purposes only and are not intended to limit the scope of this disclosure to the depicted example. In the illustrated example, the input parameter space may be partitioned according to at least two different partition schemes. In partition scheme 1210, the input parameter space may be partitioned into, for example, four regions based on the age of the glucose sensor and the sensor current Isig value of the received input measurement data, where each region of the four regions may be modeled using a respective regional SG model (e.g., model 1212, 1214, 1216, or 1218). In partition scheme 1220, the input parameter space may be partitioned into, for example, four regions based on the Vcntr value and the sensor current Isig value of the received input measurement data, where each region of the four regions may be modeled using a respective regional SG model (e.g., model 1222, 1224, 1226, or 1228).

When input data (e.g., sensor measurement data, its derivatives, and/or transformed sensor measurement data) is received, one regional SG model may be selected from models 1212, 1214, 1216, and 1218, and another regional SG model may be selected from models 1222, 1224, 1226, and 1228, based on the values of the input parameters and the two partition schemes. The outputs of the two selected regional SG models may be used in combination to generate a predicted SG value for the input data. For example, an average or weighted average of the two SG values estimated using the two selected regional SG models may be used as the predicted SG value for the input data, as described above with respect to, for example, FIG. 11.

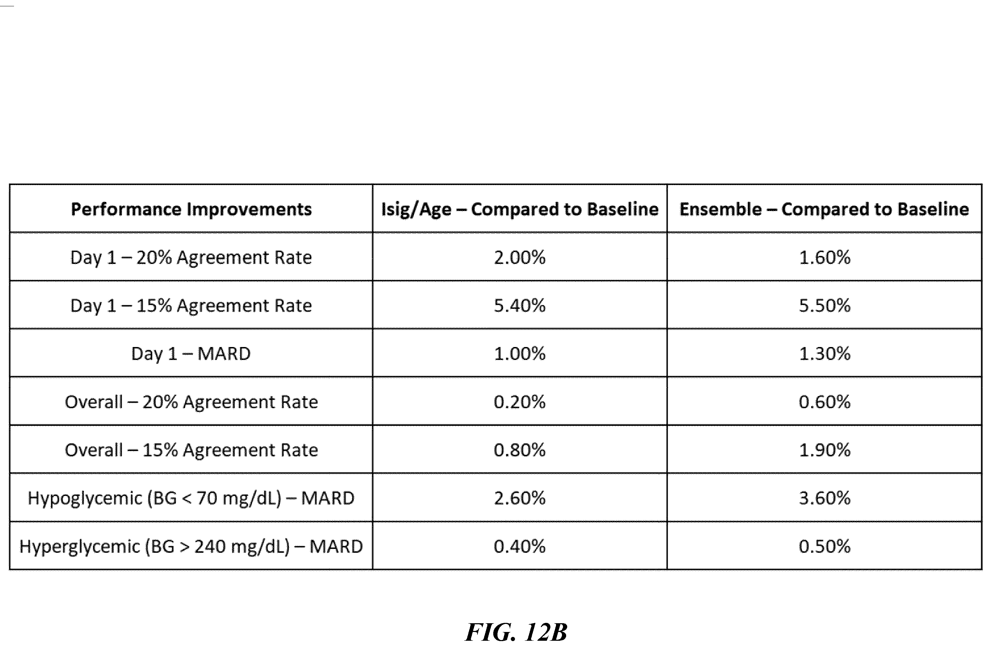

**FIG. 12B** illustrates examples of performance improvement over a baseline technique using some techniques disclosed herein according to certain embodiments. In the illustrated example, the baseline technique may use Vcntr and the age of the sensor to partition the input parameter space into four subspaces. Compared to the baseline technique, using Isig and the age of the sensor to partition the input parameter space may improve the accuracy of a CGM sensor, for example, increasing the proportion of the SG values estimated in Day 1 that are within 20% from the reference glucose levels by about 2%, increasing the proportion of the SG values estimated in Day 1 that are within 15% from the reference glucose levels by about 5.4%, reducing the MARD for Day 1 by about 1.0%, increasing the proportion of all estimated SG values that are within 20% from the reference glucose levels by about 0.2%, increasing the proportion of all estimated SG values that are within 15% from the reference glucose levels by about 0.8%, reducing the MARD for hypoglycemic conditions (e.g., BG < 70 mg/dL) by about 2.6 %, and reducing the MARD for hyperglycemic conditions (e.g., BG > 240 mg/dL) by about 0.4%.

As also shown in FIG. 12B, when an ensemble of multiple sets of regional models as disclosed herein is used, the accuracy of the CGM sensor may be further improved. For example, compared to the baseline technique, using an ensemble of multiple sets of regional models may increase the proportion of the SG values estimated in Day 1 that are within 20% from the reference glucose levels by about 1.6%, increase the proportion of the SG values estimated in Day 1 that are within 15% from the reference glucose levels by about 5.5%, reduce the MARD for Day 1 by about 1.3%, increase the proportion of all estimated SG values that are within 20% from the reference glucose levels by about 0.6%, increase the proportion of all estimated SG values that are within 15% from the reference glucose levels by about 1.9%, reduce the MARD for hypoglycemic conditions (e.g., BG < 70 mg/dL) by about 3.6 %, and reduce the MARD for hyperglycemic conditions (e.g., BG > 240 mg/dL) by about 0.5%. It is noted that examples and data illustrated in FIG. 12B are for illustration purposes only and are not intended to limit the scope of this disclosure to the illustrated examples.

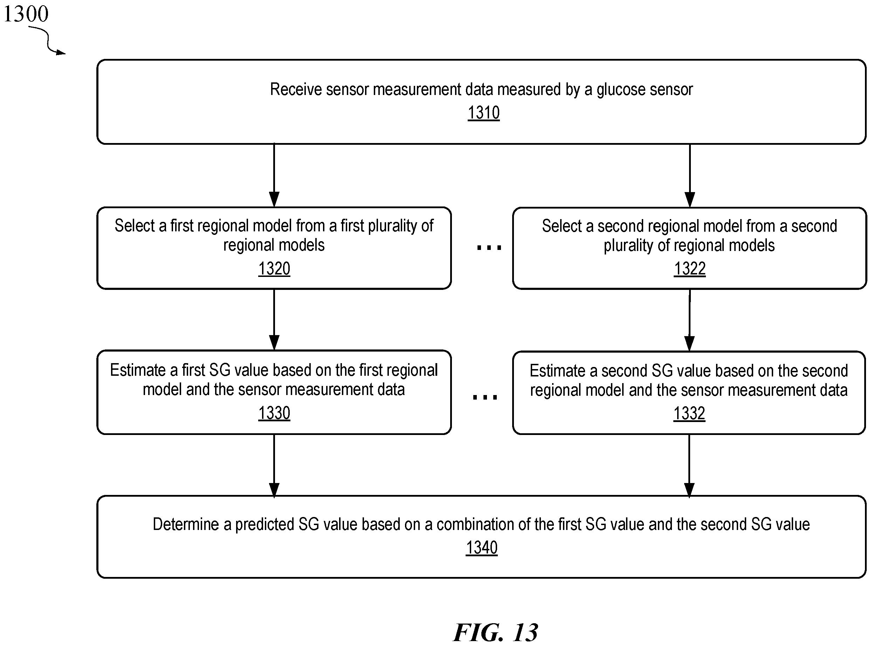

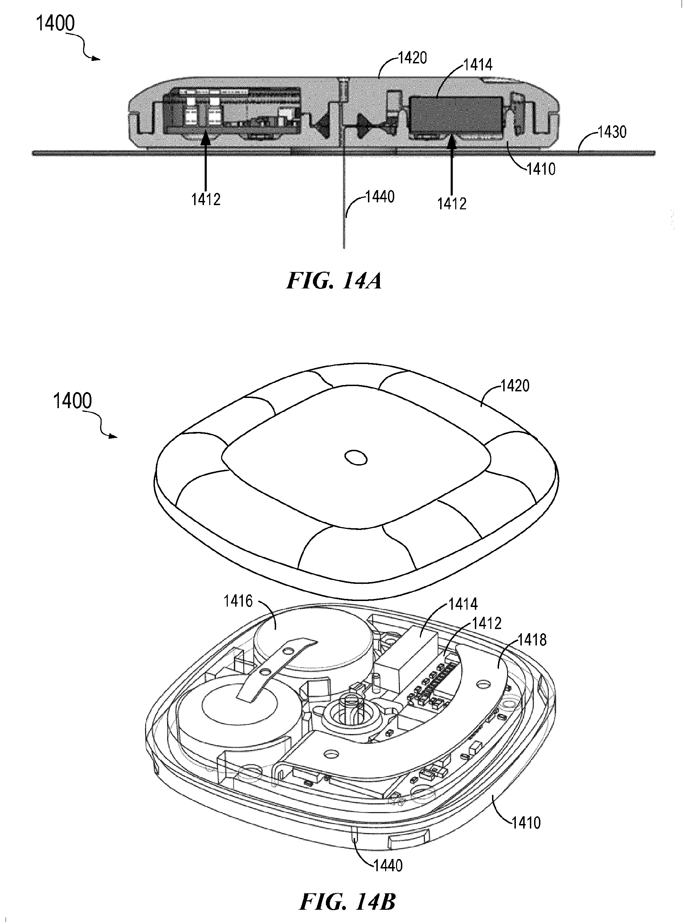

**FIG. 13** includes a flowchart 1300 illustrating an example of a process of estimating SG values using an ensemble of partitioned SG models according to certain embodiments. Operations in flowchart 1300 may be performed by, for example, monitoring device 104, glucose sensor subsystem 210, sensor electronics device 304, a glucose sensor device 1400 described below with respect to FIGS. 14A-14B, and the like. Although flowchart 1300 may describe some operations as being performed in parallel or concurrently, some of the operations may be performed in a sequential process. In addition, the order of the operations may be rearranged. The process may have additional steps not included in the figure. Some operations may be optional or may be omitted.

Operations in block 1310 may include receiving sensor measurement data measured by a glucose sensor. The sensor measurement data may include, for example, sensor current (Isig), counter voltage (Vcntr), electrochemical impedance spectroscopy (EIS) data, an age of the glucose sensor, or a combination thereof. In some embodiments, derivative features or other features may be derived, extracted, or transformed from the sensor measurement data as described above with respect to, for example, FIGS. 6-8, and may be used, in combination with the sensor measurement data, as input parameters to the SG models for estimating SG values. In some embodiments, the operations in block 1310 (or after block 1310) may include generating derivative features and/or transformed features based on the sensor measurement data.

Operations in block 1320 may include selecting, based on the sensor measurement data, a first regional SG model from a first plurality of regional SG models for respective regions of a first plurality of regions of an input parameter space associated with the sensor measurement data. The input parameter space may be partitioned into the first plurality of regions based on a first partition scheme. In some embodiments, the first partition scheme may partition the input parameter space based at least in part on a sensor current of the glucose sensor. In some embodiments, the first partition scheme may partition the input parameter space based at least in part on a sensor current and an age of the glucose sensor. Regions of the first plurality of regions of the input parameter space may be characterized by different sizes, different shapes (regular or irregular), different numbers of dimensions, or a combination thereof. In some embodiments, input parameters of the first plurality of regional SG models may include the derivative features and/or transformed features. Each regional SG model of the first plurality of regional SG models may include one or more machine learning models, equations, functions, or a combination thereof.

Operations in block 1330 may include estimating a first SG value using the first regional SG model and the sensor measurement data (and, in some embodiments, the derivative features and/or transformed features of the sensor measurement data as described above with respect to FIGS. 6-8). In some embodiments, the first regional SG model may not only estimate the first SG value, but also estimate a probability or confidence level associated with the first SG value.

Operations in block 1322 may include selecting, based on the sensor measurement data, a second regional SG model from a second plurality of regional SG models for respective regions of a second plurality of regions of the input parameter space, where the input parameter space may be partitioned into the second plurality of regions based on a second partition scheme that is different from the first partition scheme. In some embodiments, the second partition scheme may partition the input parameter space based at least in part on the sensor current of the glucose sensor. In some embodiments, the second partition scheme may partition the input parameter space based at least in part on the sensor current and the age of the glucose sensor. Regions of the second plurality of regions of the input parameter space may be characterized by different sizes, different shapes (regular or irregular), different numbers of dimensions, or a combination thereof. In some embodiments, input parameters of the second plurality of regional SG models may include the derivative features and/or transformed features. Each regional SG model of the second plurality of regional SG models may include one or more machine learning models, equations, functions, or a combination thereof.

In some embodiments, the first partition scheme and the second partition scheme may use different combinations of input parameters to partition the input parameter space. In some embodiments, the first partition scheme and the second partition scheme may use different numbers of input parameters to partition the input parameter space. In some embodiments, the first partition scheme and the second partition scheme may partition the input parameter space using different ranges of a same set of input parameters. The number of regions of the first plurality of regions of the input parameter space may be the same as or different from the number of regions of the second plurality of regions of the input parameter space.

Operations in block 1332 may include estimating a second SG value using the second regional SG model and the sensor measurement data (and, in some embodiments, the derivative features and/or transformed features of the sensor measurement data as described above with respect to FIGS. 6-8). In some embodiments, the second regional SG model may not only estimate the second SG value, but also estimate a probability or confidence level associated with the second SG value.

Operations in block 1340 may include determining a predicted SG value based on a combination of the first SG value and the second SG value. For example, determining the predicted SG value based on the combination of the first SG value and the second SG value may include determining an average or a weighted average of the first SG value and the second SG value. Weights associated with the first SG value and the second SG value for the weighted average may be pre-determined (e.g., during the learning phase) or may be determined based on, for example, the first probability or confidence level, and the second probability or confidence level.

In some embodiments, the input parameter space may be partitioned according to three or more different partition schemes, and three or more sets of regional SG models may be created for the three or more different partition schemes. One regional SG model may be selected from each respective set of regional SG models for a respective partition scheme. The predicted SG value may then be determined based on outputs of the selected three or more regional SG models. For example, in some embodiments, operations of flowchart 1300 may also include selecting, based on the sensor measurement data, a third regional SG model from a third plurality of regional SG models for respective regions of a third plurality of regions of the input parameter space, where the input parameter space may be partitioned into the third plurality of regions based on a third partition scheme that is different from the first partition scheme and the second partition scheme; and estimating a third SG value using the third regional SG model and the sensor measurement data, where determining the predicted SG value may include determining the predicted SG value based on a combination (e.g., an average or weighted average) of the first SG value, the second SG value, and the third SG value.

**FIGS. 14A** and **14B** depict an example of an integrated glucose sensor device 1400 according to certain embodiments, which may implement some of the examples disclosed herein. Glucose sensor device 1400 may be an example of monitoring device 104, glucose sensor subsystem 210, or CGM system 300. In the illustrated example, glucose sensor device 1400 may include a housing formed by a base 1410 and a top cover 1420, sensor electronics enclosed in the housing, an electrochemical sensor 1440 partially within the housing and connected to the sensor electronics, and an adhesive patch 1430 attached to base 1410. Electrochemical sensor 1440 may be an example of the electrochemical sensors described above (e.g., glucose sensor 400 or 402), and may include multiple electrodes at a distal end that may be inserted into the subcutaneous layer of a user. A proximal end of electrochemical sensor 1440 may be bent and held against a printed circuit board (PCB) 1412 by a elastomer or rubber block 1414, such that contacts on the proximal end of electrochemical sensor 1440 may be physically and electrically connected to contact pads on PCB 1412. The housing may be hermetically sealed to protect the sensor electronics from moisture. Electrochemical sensor 1440 may be inserted into the subcutaneous layer of the user using an inserter. The inserter may include a needle and may enclose the entire glucose sensor device 1400 before insertion. When activated (e.g., pushed), the inserter may move the entire glucose sensor device 1400 toward the skin of the user to attach adhesive patch 1430 to the skin of the user and insert the distal end of electrochemical sensor 1440 into the subcutaneous layer of the user using the needle, where the needle may be retracted, for example, automatically after the distal end of electrochemical sensor 1440 is inserted into the user.

The sensor electronics in the housing may include batteries 1416, an antenna 1418, and circuits on PCB 1412. The circuits on PCB 1412 may include, for example, a processor, a controller, a potentiostat, filters, amplifiers, a wireless (e.g., Bluetooth) transceiver, one or more memory devices, power conversion and management devices, or a combination thereof. Predicted in vivo characteristics of electrochemical sensor 1440 and SG model(s) described above may be stored in the one or more memory devices. Before or after the distal end of electrochemical sensor 1440 is inserted into the subcutaneous layer of the user, the sensor electronics may be activated to initialize the sensor for measuring sensor measurement data (e.g., Isig, Vcntr, EIS, etc.). The processor or controller may execute instruction code stored on the one or more memory device to obtain the sensor measurement data, process (e.g., filter, amplify, transform, etc.) the sensor measurement data, estimate SG values using the SG models and sensor measurement data, optionally blank certain estimated SG values, and transmit, via the wireless transceiver and antenna 1418, the estimated SG values to a receiver (e.g., a smartphone or an insulin pump), as described above.

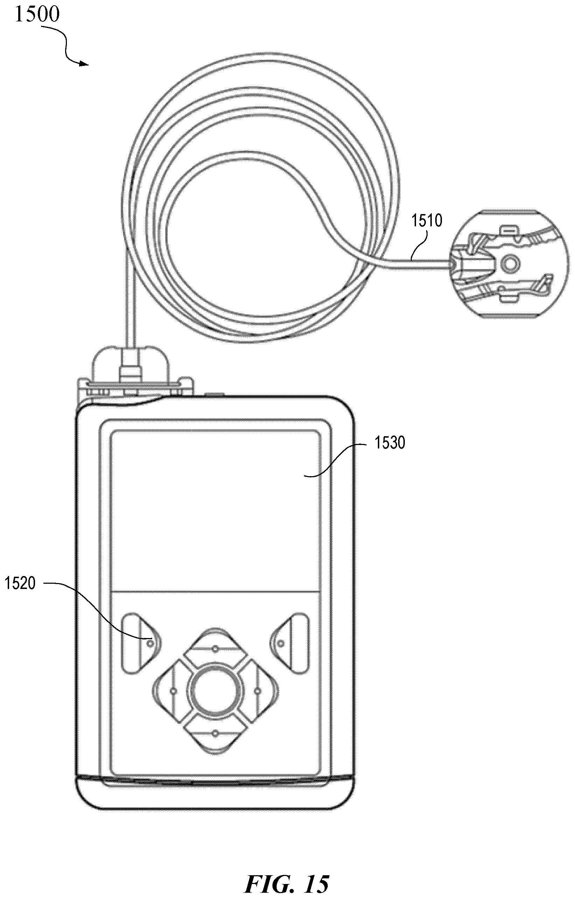

**FIG. 15** depicts an example of a delivery device 1500, which may implement a delivery device described above (e.g., delivery device 102 or insulin delivery subsystem 230). In an insulin delivery device implemented using delivery device 1500, insulin delivery may be performed based on internal communication between a central computing module (e.g., a microprocessor of delivery device 1500) and an insulin delivery module (e.g., including a microcontroller, a motor, and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., computing device 106 of FIG. 1) communicatively coupled to a remote or cloud computing system (e.g., remote/cloud computing system 108 of FIG. 1). Delivery device 1500 may communicate various event data toward the remote or cloud computing system, which may communicate insulin delivery determinations toward delivery device 1500, in accordance with the techniques described herein.

Delivery device 1500 may provide insulin through a small tube 1510 configured for fluidic connection with a cannula inserted subcutaneously. Delivery device 1500 may be configured to deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. In the depicted example, delivery device 1500 includes a user interface having button elements 1520 that can be manipulated to administer a bolus of insulin, to change therapy settings, to change user preferences, to select display features, and the like. Delivery device 1500 may also include a display device 1530 that can be used to present various types of information or data to the user. In accordance with aspects of the present disclosure, a user of delivery device 1500 may use button elements 1520 to input certain event data (e.g., event type, event start time, event details, etc.), and the user inputs can be confirmed using display device 1530. Delivery device 1500 of FIG. 15 is merely provided by way of example, and other types of insulin delivery devices and other techniques different from those described above are contemplated to be within the scope of the present disclosure.

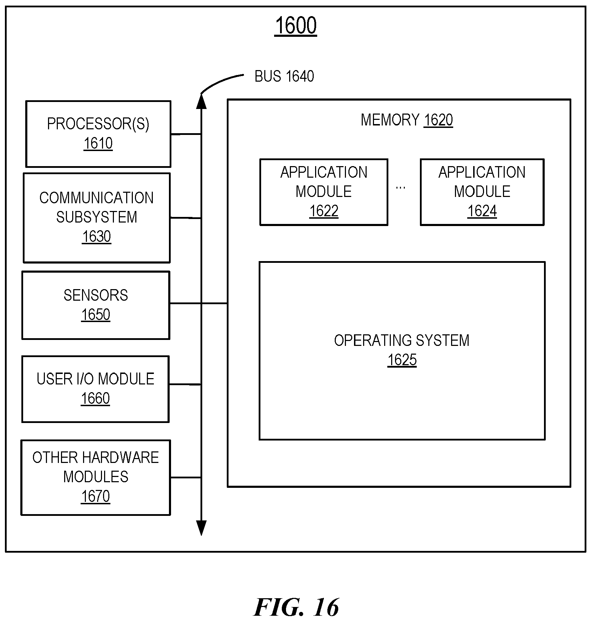

**FIG. 16** is a block diagram of an example of an electronic device 1600 that may implement some of the examples disclosed herein. For example, electronic device 1600 may be used to implement delivery device 102, monitoring device 104, computing device 106, and remote/cloud computing system 108, glucose sensor subsystem 210, controller 220, insulin delivery subsystem 230, glucose delivery subsystem 240, and glucagon delivery subsystem 250, CGM system 300, glucose sensor device 1400, delivery device 1500, or another device that may be used to calculate insulin dose and/or predict future blood glucose levels. In the illustrated example, electronic device 1600 may include one or more processor(s) 1610 and memory 1620. Processor(s) 1610 may be configured to execute instructions stored in memory 1620 for performing one or more of the methods described herein and other applications. Processor(s) 1610 may include, for example, one or more central processing units, microprocessors, microcontrollers, special-purpose processors (e.g., digital signal processors), ASICs, DSPs, FPGAs, or other processors suitable for implementation within a portable electronic device. Processor(s) 1610 may be communicatively coupled with a plurality of components within electronic device 1600. To realize this communicative coupling, processor(s) 1610 may communicate with the other illustrated components across a bus 1640. Bus 1640 may be any subsystem adapted to transfer data within electronic device 1600. Bus 1640 may include a plurality of computer buses and additional circuitry to transfer data.

Memory 1620 may include one or more transitory and/or non-transitory storage devices, such as, for example, a static random-access memory (SRAM), a dynamic random access memory (DRAM), a read-access memory (RAM), a read-only memory (ROM), a programmable read-only memory (PROM), an erasable programmable read-only memory (EPROM), a FLASH-EPROM, a secure digital (SD) card, and any other memory chip or cartridge. Such storage devices may be configured to implement any appropriate data stores, including without limitation, various file systems, data structures, computer-readable instructions, program modules, and the like. In some embodiments, memory 1620 may be distributed into different hardware modules.

Memory 1620 may include an operating system 1625 loaded therein. Operating system 1625 may be operable to initiate the execution of the instructions provided by application modules 1622-1824 and/or manage other hardware modules 1670, as well as interface with a communication subsystem 1630 which may include one or more wired and/or wireless transceivers. Operating system 1625 may be adapted to perform other operations across the components of electronic device 1600 including threading, virtualization, resource management, data storage control, and other similar functionality. In some embodiments, memory 1620 may store a plurality of application modules 1622 through 1624, which may include any number of applications. Examples of the applications may include an insulin calculator, a blood glucose level monitor or predictor, a glucose level management application, and the like. Application modules 1622-1424 may include particular instructions to be executed by processor(s) 1610. In some embodiments, certain applications or parts of application modules 1622-1424 may be executable by other hardware modules 1670.

Communication subsystem 1630 may include, for example, an infrared communication device, a wireless communication device and/or chipset (such as a Bluetooth® device, an IEEE 802.11 device, a Wi-Fi device, a WiMax device, cellular communication devices, etc.), and/or similar communication interfaces. One or more antennas (not shown) may be used for wireless communication as part of communication subsystem 1630 or as a separate component coupled to any portion of electronic device 1600, such as a wireless charging receiver or a near-field communication receiver. In some embodiments, communication subsystem 1630 may include circuits for wired communication technologies, such as Ethernet, coaxial communications, universal serial bus (USB), and the like. Communications subsystem 1630 may permit data to be exchanged with a network, other computer systems, and/or any other devices. For example, communications subsystem 1630 may be used to receive therapy determinations for therapeutic fluid (e.g., insulin) delivery, such as from a cloud computing system via an intermediary computing device (e.g., a controller) communicatively coupled to electronic device 1600, where processor(s) 1610 may, based on the therapy determinations, send commands to an actuator controller to cause the delivery of appropriate amounts of therapeutic fluid (e.g., insulin) to a user. In another example, communications subsystem 1630 may be used to communicate measurement results (e.g., sensor glucose levels) to a computing device (e.g., a smartphone or a personal health monitoring device) and/or to a remote server via the computing device, or receive data (e.g., calibration data, configuration data, etc.) from the computing device or the remote server via the computing device.

Sensor(s) 1650 may include, for example, a camera, an infrared sensor, an accelerometer, a pressure sensor, a temperature sensor, a proximity sensor, a magnetometer, a gyroscope, an inertial sensor (e.g., an inertial measurement unit (IMU)), an ambient light sensor, a position sensor, a depth sensor, a gesture detector, or any other similar module operable to provide sensory output and/or receive sensory input. In one example, sensor(s) 1650 may be used to implement a meal detector.

Input/output user interface 1660 may allow a user to send action requests to electronic device 1600 to perform particular actions, and may provide information (e.g., status of electronic device 1600, measurement results, alerts, etc.) to the user. Input/output user interface 1660 may include one or more input devices, such as, for example, a touchscreen, a touch pad, microphone(s), button(s), dial(s), switch(es), a keyboard, a mouse, a game controller, or any other suitable device for receiving action requests and communicating the received action requests to processor(s) 1610. In some embodiments, input/output user interface 1660 may include one or more output devices, such as a display, a speaker, a light emitting device, a haptic device, and the like, to provide feedback or alarm to the user.

In some embodiments, electronic device 1600 may include a plurality of other hardware modules 1670. Each of other hardware modules 1670 may be a physical module within electronic device 1600. While each of other hardware modules 1670 may be permanently configured as a structure, some of other hardware modules 1670 may be temporarily configured to perform specific functions or temporarily activated. Examples of other hardware modules 1670 may include, for example, an audio output and/or input module (e.g., a microphone or speaker), a near field communication (NFC) module, a rechargeable battery, a battery management system, a wired/wireless battery charging system, an actuator controller, and the like. In some embodiments, one or more functions of other hardware modules 1670 may be implemented in software.

In one example, electronic device 1600 may be part of an insulin delivery device (e.g., a pump) that can deliver fast-acting insulin through a small tube configured for fluidic connection with a cannula inserted subcutaneously. Electronic device 1600 may cause the delivery of two types of dosages-a basal dosage, which can be delivered periodically (e.g., every five minutes) in tiny amounts throughout the day and night, and a bolus dosage to cover an increase in blood glucose from meals and/or to correct high blood glucose levels. The insulin delivery device may include a user interface having button elements that can be manipulated to administer a bolus of insulin, to change therapy settings, to change user preferences, to select display features, and the like. The insulin delivery device may also include a display device that can be used to present various types of information or data to the user. In accordance with aspects of the present disclosure, a user of the insulin delivery device may use the button elements to input certain event data (e.g., event type, event start time, event details, etc.), and the user inputs can be confirmed using the display device.

In various implementations, the above-described hardware and modules may be implemented on a single device or on multiple devices that can communicate with one another using wired or wireless connections. In alternative configurations, different and/or additional components may be included in electronic device 1600. Similarly, functionality of one or more of the components can be distributed among the components in a manner different from the manner described above.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the spirit and scope of the present disclosure.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure. While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto. The aspects and features of the present disclosure and may be embodied in various forms. Thus, specific structural and functional details disclosed herein are not to be interpreted as limiting, but as a basis for the claims and as a representative basis for teaching one skilled in the art to variously employ the present disclosure in any appropriately detailed structure.

With reference to the appended figures, components that can include memory can include non-transitory machine-readable media. The term "machine-readable medium," "processor-readable medium," or "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of' if used to associate a list, such as A, B, or C, can be interpreted to mean A, B, C, or any combination of A, B, and/or C, such as AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for inter-process communications, and different pairs of processes may use different techniques, or the same pair of processes may use different techniques at different times.

In view of this description embodiments may include different combinations of features. Implementation examples are described in the following numbered clauses:

**Clause** 1. A processor-implemented method comprising:

receiving sensor measurement data measured by a glucose sensor, the sensor measurement data including at least one signal that is indicative of glucose level and at least one signal that is indicative of sensor health; selecting, based at least in part on the sensor measurement data, a first regional sensor glucose (SG) model from a first plurality of regional SG models for respective regions of a first plurality of regions of an input parameter space associated with the sensor measurement data, wherein the input parameter space is partitioned into the first plurality of regions based on a first partition scheme; estimating a first SG value using the first regional SG model and the sensor measurement data; selecting, based at least in part on the sensor measurement data, a second regional SG model from a second plurality of regional SG models for respective regions of a second plurality of regions of the input parameter space, wherein the input parameter space is partitioned into the second plurality of regions based on a second partition scheme that is different from the first partition scheme;

estimating a second SG value using the second regional SG model and the sensor measurement data; and

determining a predicted SG value based on a combination of the first SG value and the second SG value.

**Clause 2.** The processor-implemented method of Clause 1, wherein at least one of the first partition scheme or the second partition scheme partitions the input parameter space based at least in part on sensor current (Isig), counter voltage (Vcntr), electrochemical impedance spectroscopy (EIS) data, age of the glucose sensor, temperature, age of a user of the glucose sensor, body mass index (BMI) of the user, or a combination thereof.

**Clause 3.** The processor-implemented method of Clause 1 or 2, wherein the first partition scheme or the second partition scheme partitions the input parameter space based at least in part on a sensor current and an age of the glucose sensor.

**Clause 4.** The processor-implemented method of any of Clauses 1-3, wherein the first partition scheme and the second partition scheme use different combinations of input parameters to partition the input parameter space.

**Clause 5.** The processor-implemented method of any of Clauses 1-4, wherein the first partition scheme and the second partition scheme use different numbers of input parameters to partition the input parameter space.

**Clause 6.** The processor-implemented method of any of Clauses 1-5, wherein the first partition scheme and the second partition scheme partition the input parameter space using different ranges of a same set of input parameters.

**Clause 7.** The processor-implemented method of any of Clauses 1-6, wherein regions of the first plurality of regions of the input parameter space are characterized by different sizes, different shapes, different numbers of dimensions, or a combination thereof.

**Clause 8.** The processor-implemented method of any of Clauses 1-7, wherein a number of regions of the first plurality of regions of the input parameter space is the same as or different from a number of regions of the second plurality of regions of the input parameter space.

**Clause 9.** The processor-implemented method of any of Clauses 1-8, further comprising generating at least one of a derivative feature or a transformed feature based on the sensor measurement data, wherein input parameters of the first plurality of regional SG models and the second plurality of regional SG models include at least one of the derivative feature or the transformed feature.

**Clause 10.** The processor-implemented method of any of Clauses 1-9, wherein determining the predicted SG value based on the combination of the first SG value and the second SG value comprises determining an average or a weighted average of the first SG value and the second SG value.

**Clause 11.** The processor-implemented method of Clause 10, further comprising:

determining a first probability or confidence level associated with the first SG value; and

determining a second probability or confidence level associated with the first SG value,

wherein weights associated with the first SG value and the second SG value for the weighted average are determined based on the first probability or confidence level, and the second probability or confidence level.

**Clause 12.** The processor-implemented method of any of Clauses 1-11, further comprising:

selecting, based at least in part on the sensor measurement data, a third regional SG model from a third plurality of regional SG models for respective regions of a third plurality of regions of the input parameter space, wherein the input parameter space is partitioned into the third plurality of regions based on a third partition scheme that is different from the first partition scheme and the second partition scheme; and

estimating a third SG value using the third regional SG model and the sensor measurement data,

wherein determining the predicted SG value includes determining the predicted SG value based on a combination of the first SG value, the second SG value, and the third SG value.

**Clause 13.** The processor-implemented method of any of Clauses 1-12, wherein:

each regional SG model of the first plurality of regional SG models and the second plurality of regional SG models includes one or more machine learning models, equations, functions, or a combination thereof; and

a first regional SG model and a second regional SG model in the first plurality of regional SG models and the second plurality of regional SG models use different input parameters.

**Clause 14.** A system comprising:

one or more processors; and

one or more processor-readable media storing instructions which, when executed by the one or more processors, cause performance of operations including:

receiving sensor measurement data measured by a glucose sensor, the sensor measurement data including at least one signal that is indicative of glucose level and at least one signal that is indicative of sensor health; selecting, based at least in part on the sensor measurement data, a first regional sensor glucose (SG) model from a first plurality of regional SG models for respective regions of an input parameter space associated with the sensor measurement data, wherein the input parameter space is partitioned into the first plurality of regions based on a first partition scheme; estimating a first SG value using the first regional SG model and the sensor measurement data; selecting, based at least in part on the sensor measurement data, a second regional SG model from a second plurality of regional SG models for respective regions of the input parameter space, wherein the input parameter space is partitioned into the second plurality of regions based on a second partition scheme that is different from the first partition scheme; estimating a second SG value using the second regional SG model and the sensor measurement data; and determining a predicted SG value based on a combination of the first SG value and the second SG value.

**Clause 15.** One or more non-transitory processor-readable media storing instructions which, when executed by one or more processors, cause performance of operations comprising:

receiving sensor measurement data measured by a glucose sensor, the sensor measurement data including at least one signal that is indicative of glucose level and at least one signal that is indicative of sensor health; selecting, based at least in part on the sensor measurement data, a first regional sensor glucose (SG) model from a first plurality of regional SG models for respective regions of an input parameter space associated with the sensor measurement data, wherein the input parameter space is partitioned into the first plurality of regions based on a first partition scheme; estimating a first SG value using the first regional SG model and the sensor measurement data; selecting, based at least in part on the sensor measurement data, a second regional SG model from a second plurality of regional SG models for respective regions of the input parameter space, wherein the input parameter space is partitioned into the second plurality of regions based on a second partition scheme that is different from the first partition scheme; estimating a second SG value using the second regional SG model and the sensor measurement data; and determining a predicted SG value based on a combination of the first SG value and the second SG value.

Further disclosed herein is the subject-matter of the following items:

1. A processor-implemented method comprising:

receiving sensor measurement data measured by a glucose sensor, the sensor measurement data including at least one signal that is indicative of glucose level and at least one signal that is indicative of sensor health; selecting, based at least in part on the sensor measurement data, a first regional sensor glucose (SG) model from a first plurality of regional SG models for respective regions of a first plurality of regions of an input parameter space associated with the sensor measurement data, wherein the input parameter space is partitioned into the first plurality of regions based on a first partition scheme; estimating a first SG value using the first regional SG model and the sensor measurement data; selecting, based at least in part on the sensor measurement data, a second regional SG model from a second plurality of regional SG models for respective regions of a second plurality of regions of the input parameter space, wherein the input parameter space is partitioned into the second plurality of regions based on a second partition scheme that is different from the first partition scheme; estimating a second SG value using the second regional SG model and the sensor measurement data; and determining a predicted SG value based on a combination of the first SG value and the second SG value.

2. The processor-implemented method of item 1, wherein at least one of the first partition scheme or the second partition scheme partitions the input parameter space based at least in part on sensor current (Isig), counter voltage (Vcntr), electrochemical impedance spectroscopy (EIS) data, age of the glucose sensor, temperature, age of a user of the glucose sensor, body mass index (BMI) of the user, or a combination thereof.

3. The processor-implemented method of item 1 or 2, wherein the first partition scheme or the second partition scheme partitions the input parameter space based at least in part on a sensor current and an age of the glucose sensor.

4. The processor-implemented method of item 1 or of one of items 1 to 3, wherein the first partition scheme and the second partition scheme use different combinations of input parameters to partition the input parameter space.

5. The processor-implemented method of item 1 or of one of items 1 to 4, wherein the first partition scheme and the second partition scheme use different numbers of input parameters to partition the input parameter space.

6. The processor-implemented method of item 1 or of one of items 1 to 5, wherein the first partition scheme and the second partition scheme partition the input parameter space using different ranges of a same set of input parameters.

7. The processor-implemented method of item 1 or of one of items 1 to 6, wherein regions of the first plurality of regions of the input parameter space are characterized by different sizes, different shapes, different numbers of dimensions, or a combination thereof.

8. The processor-implemented method of item 1 or of one of items 1 to 7, wherein a number of regions of the first plurality of regions of the input parameter space is the same as or different from a number of regions of the second plurality of regions of the input parameter space.

9. The processor-implemented method of item 1 or of one of items 1 to 8, further comprising generating at least one of a derivative feature or a transformed feature based on the sensor measurement data, wherein input parameters of the first plurality of regional SG models and the second plurality of regional SG models include at least one of the derivative feature or the transformed feature.

10. The processor-implemented method of item 1 or of one of items 1 to 9, wherein determining the predicted SG value based on the combination of the first SG value and the second SG value comprises determining an average or a weighted average of the first SG value and the second SG value.

11. The processor-implemented method of item 10, further comprising:

determining a first probability or confidence level associated with the first SG value; and
determining a second probability or confidence level associated with the first SG value,
wherein weights associated with the first SG value and the second SG value for the weighted average are determined based on the first probability or confidence level, and the second probability or confidence level.

12. The processor-implemented method of item 1 or of one of items 1 to 11, further comprising:

selecting, based at least in part on the sensor measurement data, a third regional SG model from a third plurality of regional SG models for respective regions of a third plurality of regions of the input parameter space, wherein the input parameter space is partitioned into the third plurality of regions based on a third partition scheme that is different from the first partition scheme and the second partition scheme; and
estimating a third SG value using the third regional SG model and the sensor measurement data,
wherein determining the predicted SG value includes determining the predicted SG value based on a combination of the first SG value, the second SG value, and the third SG value.

13. The processor-implemented method of item 1 or of one of items 1 to 12, wherein:

each regional SG model of the first plurality of regional SG models and the second plurality of regional SG models includes one or more machine learning models, equations, functions, or a combination thereof; and
a first regional SG model and a second regional SG model in the first plurality of regional SG models and the second plurality of regional SG models use different input parameters.

14. A system comprising:

one or more processors; and
one or more processor-readable media storing instructions which, when executed by the one or more processors, cause performance of operations including:

receiving sensor measurement data measured by a glucose sensor, the sensor measurement data including at least one signal that is indicative of glucose level and at least one signal that is indicative of sensor health;

selecting, based at least in part on the sensor measurement data, a first regional sensor glucose (SG) model from a first plurality of regional SG models for respective regions of an input parameter space associated with the sensor measurement data, wherein the input parameter space is partitioned into the first plurality of regions based on a first partition scheme;

estimating a first SG value using the first regional SG model and the sensor measurement data;

selecting, based at least in part on the sensor measurement data, a second regional SG model from a second plurality of regional SG models for respective regions of the input parameter space, wherein the input parameter space is partitioned into the second plurality of regions based on a second partition scheme that is different from the first partition scheme;

estimating a second SG value using the second regional SG model and the sensor measurement data; and

determining a predicted SG value based on a combination of the first SG value and the second SG value.

15. One or more non-transitory processor-readable media storing instructions which, when executed by one or more processors, cause performance of operations comprising:

receiving sensor measurement data measured by a glucose sensor, the sensor measurement data including at least one signal that is indicative of glucose level and at least one signal that is indicative of sensor health;

selecting, based at least in part on the sensor measurement data, a first regional sensor glucose (SG) model from a first plurality of regional SG models for respective regions of an input parameter space associated with the sensor measurement data, wherein the input parameter space is partitioned into the first plurality of regions based on a first partition scheme;

estimating a first SG value using the first regional SG model and the sensor measurement data;

selecting, based at least in part on the sensor measurement data, a second regional SG model from a second plurality of regional SG models for respective regions of the input parameter space, wherein the input parameter space is partitioned into the second plurality of regions based on a second partition scheme that is different from the first partition scheme;

estimating a second SG value using the second regional SG model and the sensor measurement data; and

determining a predicted SG value based on a combination of the first SG value and the second SG value.

**Claims**

1.  A processor-implemented method comprising:

receiving sensor measurement data measured by a glucose sensor, the sensor measurement data including at least one signal that is indicative of glucose level and at least one signal that is indicative of sensor health;

selecting, based at least in part on the sensor measurement data, a first regional sensor glucose (SG) model from a first plurality of regional SG models for respective regions of a first plurality of regions of an input parameter space associated with the sensor measurement data, wherein the input parameter space is partitioned into the first plurality of regions based on a first partition scheme;

estimating a first SG value using the first regional SG model and the sensor measurement data;

selecting, based at least in part on the sensor measurement data, a second regional SG model from a second plurality of regional SG models for respective regions of a second plurality of regions of the input parameter space, wherein the input parameter space is partitioned into the second plurality of regions based on a second partition scheme that is different from the first partition scheme;

estimating a second SG value using the second regional SG model and the sensor measurement data; and

determining a predicted SG value based on a combination of the first SG value and the second SG value.

2.  The processor-implemented method of claim 1, wherein at least one of the first partition scheme or the second partition scheme partitions the input parameter space based at least in part on sensor current (Isig), counter voltage (Vcntr), electrochemical impedance spectroscopy (EIS) data, age of the glucose sensor, temperature, age of a user of the glucose sensor, body mass index (BMI) of the user, or a combination thereof.

3.  The processor-implemented method of claim 1 or 2, wherein the first partition scheme or the second partition scheme partitions the input parameter space based at least in part on a sensor current and an age of the glucose sensor.

4.  The processor-implemented method of one of claims 1 to 3, wherein the first partition scheme and the second partition scheme use different combinations of input parameters to partition the input parameter space.

5. The processor-implemented method of one of claims 1 to 4, wherein the first partition scheme and the second partition scheme use different numbers of input parameters to partition the input parameter space.

6. The processor-implemented method of one of claims 1 to 5, wherein the first partition scheme and the second partition scheme partition the input parameter space using different ranges of a same set of input parameters.

7. The processor-implemented method of one of claims 1 to 6, wherein regions of the first plurality of regions of the input parameter space are **characterized by** different sizes, different shapes, different numbers of dimensions, or a combination thereof.

8. The processor-implemented method of one of claims 1 to 7, wherein a number of regions of the first plurality of regions of the input parameter space is the same as or different from a number of regions of the second plurality of regions of the input parameter space.

9. The processor-implemented method of one of claims 1 to 8, further comprising generating at least one of a derivative feature or a transformed feature based on the sensor measurement data, wherein input parameters of the first plurality of regional SG models and the second plurality of regional SG models include at least one of the derivative feature or the transformed feature.

10. The processor-implemented method of one of claims 1 to 9, wherein determining the predicted SG value based on the combination of the first SG value and the second SG value comprises determining an average or a weighted average of the first SG value and the second SG value.

11. The processor-implemented method of claim 10, further comprising:

   determining a first probability or confidence level associated with the first SG value; and
   determining a second probability or confidence level associated with the first SG value,
   wherein weights associated with the first SG value and the second SG value for the weighted average are determined based on the first probability or confidence level, and the second probability or confidence level.

12. The processor-implemented method of one of claims 1 to 11, further comprising:

   selecting, based at least in part on the sensor measurement data, a third regional SG model from a third plurality of regional SG models for respective regions of a third plurality of regions of the input parameter space, wherein the input parameter space is partitioned into the third plurality of regions based on a third partition scheme that is different from the first partition scheme and the second partition scheme; and
   estimating a third SG value using the third regional SG model and the sensor measurement data,
   wherein determining the predicted SG value includes determining the predicted SG value based on a combination of the first SG value, the second SG value, and the third SG value.

13. The processor-implemented method of one of claims 1 to 12, wherein:

   each regional SG model of the first plurality of regional SG models and the second plurality of regional SG models includes one or more machine learning models, equations, functions, or a combination thereof; and
   a first regional SG model and a second regional SG model in the first plurality of regional SG models and the second plurality of regional SG models use different input parameters.

14. A system comprising:

   one or more processors; and
   one or more processor-readable media storing instructions which, when executed by the one or more processors, cause performance of operations including:

      receiving sensor measurement data measured by a glucose sensor, the sensor measurement data including at least one signal that is indicative of glucose level and at least one signal that is indicative of sensor health;
      selecting, based at least in part on the sensor measurement data, a first regional sensor glucose (SG) model from a first plurality of regional SG models for respective regions of an input parameter space associated with the sensor measurement data, wherein the input parameter space is partitioned into the first plurality of regions based on a first partition scheme;

estimating a first SG value using the first regional SG model and the sensor measurement data;

selecting, based at least in part on the sensor measurement data, a second regional SG model from a second plurality of regional SG models for respective regions of the input parameter space, wherein the input parameter space is partitioned into the second plurality of regions based on a second partition scheme that is different from the first partition scheme;

estimating a second SG value using the second regional SG model and the sensor measurement data; and

determining a predicted SG value based on a combination of the first SG value and the second SG value.

**15.** One or more non-transitory processor-readable media storing instructions which, when executed by one or more processors, cause performance of operations comprising:

receiving sensor measurement data measured by a glucose sensor, the sensor measurement data including at least one signal that is indicative of glucose level and at least one signal that is indicative of sensor health;

selecting, based at least in part on the sensor measurement data, a first regional sensor glucose (SG) model from a first plurality of regional SG models for respective regions of an input parameter space associated with the sensor measurement data, wherein the input parameter space is partitioned into the first plurality of regions based on a first partition scheme;

estimating a first SG value using the first regional SG model and the sensor measurement data;

selecting, based at least in part on the sensor measurement data, a second regional SG model from a second plurality of regional SG models for respective regions of the input parameter space, wherein the input parameter space is partitioned into the second plurality of regions based on a second partition scheme that is different from the first partition scheme;

estimating a second SG value using the second regional SG model and the sensor measurement data; and

determining a predicted SG value based on a combination of the first SG value and the second SG value.

*FIG. 1*

FOOD INTAKE

215 — MEAL INTAKE MONITOR/ DETECTOR

220 — CONTROLLER

COMMANDS

SENSOR GLUCOSE

210 — GLUCOSE SENSOR

250 — GLUCAGON DELIVERY

240 — GLUCOSE DELIVERY

230 — INSULIN DELIVERY

GLUCAGON

GLUCOSE

INSULIN

BLOOD GLUCOSE LEVEL

260 — BODY

200

*FIG. 2*

DISPLAY | MONITOR

FIG. 3

EP 4 545 002 A1

**FIG. 4A**

**FIG. 4B**

**FIG. 4C**

FIG. 5

*FIG. 6*

EP 4 545 002 A1

**FIG. 7**

**FIG. 8**

FIG. 9B

FIG. 9A

40

*FIG. 10A*

*FIG. 10B*

**FIG. 11**

EP 4 545 002 A1

**FIG. 12A**

EP 4 545 002 A1

| Performance Improvements | Isig/Age – Compared to Baseline | Ensemble – Compared to Baseline |
|---|---|---|
| Day 1 – 20% Agreement Rate | 2.00% | 1.60% |
| Day 1 – 15% Agreement Rate | 5.40% | 5.50% |
| Day 1 – MARD | 1.00% | 1.30% |
| Overall – 20% Agreement Rate | 0.20% | 0.60% |
| Overall – 15% Agreement Rate | 0.80% | 1.90% |
| Hypoglycemic (BG < 70 mg/dL) – MARD | 2.60% | 3.60% |
| Hyperglycemic (BG > 240 mg/dL) – MARD | 0.40% | 0.50% |

*FIG. 12B*

EP 4 545 002 A1

1300

Receive sensor measurement data measured by a glucose sensor
1310

Select a first regional model from a first plurality of regional models
1320

. . .

Select a second regional model from a second plurality of regional models
1322

Estimate a first SG value based on the first regional model and the sensor measurement data
1330

. . .

Estimate a second SG value based on the second regional model and the sensor measurement data
1332

Determine a predicted SG value based on a combination of the first SG value and the second SG value
1340

*FIG. 13*

EP 4 545 002 A1

**FIG. 14A**

**FIG. 14B**

1500

1510

1530

1520

**FIG. 15**

**FIG. 16**

EP 4 545 002 A1

# EP 4 545 002 A1

Europäisches
Patentamt

European
Patent Office

Office européen
des brevets

# EUROPEAN SEARCH REPORT

Application Number

EP 24 20 8161

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/233108 A1 (AJEMBA PETER [US] ET AL) 28 July 2022 (2022-07-28)<br>* figures 4,5,7,8 *<br>* paragraphs [0025], [0050] - [0054] *<br>* claim 15 *<br>- - - - - | 1-15 | INV.<br>A61B5/145<br>A61B5/00<br><br>ADD.<br>A61B5/1455<br>A61B5/1495 |

TECHNICAL FIELDS
SEARCHED       (IPC)

A61B

The present search report has been drawn up for all claims

| Place of search | Date of completion of the search | Examiner |
|---|---|---|
| The Hague | 7 March 2025 | Albrecht, Ronald |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

49

**EP 4 545 002 A1**

## ANNEX TO THE EUROPEAN SEARCH REPORT
## ON EUROPEAN PATENT APPLICATION NO.

EP 24 20 8161

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2025

| Patent document cited in search report | Publication date | Patent family member(s) | Publication date |
|---|---|---|---|
| US 2022233108 A1 | 28-07-2022 | US 2022233108 A1<br>US 2022233109 A1 | 28-07-2022<br>28-07-2022 |

EPO FORM P0459

For more details about this annex : see Official Journal of the European Patent Office, No. 12/82

**REFERENCES CITED IN THE DESCRIPTION**

**Patent documents cited in the description**

- US 773422